# EUROPEAN PATENT APPLICATION

(11) **EP 4 458 853 A1**
(43) Date of publication of application: **06.11.2024**
(21) Application number: 22915091.7
(22) Date of filing: 29.12.2022
(51) Int. Cl.: C07K 16/28, C12N 15/13, C12N 15/63, C12N 5/10, A61K 39/395, A61P 35/00, G01N 33/68, G01N 33/574

(54) **DEVELOPMENT OF NOVEL UPAR SINGLE-DOMAIN ANTIBODY**

(30) Priority: 29.12.2021 CN 202111642915
(71) Applicant: Persongen Biotherapeutics (Suzhou) Co., Ltd., Suzhou, Jiangsu 215000 (CN)
(72) Inventor: YANG, Lin, Suzhou, Jiangsu 215000 (CN); YOU, Fengtao, Suzhou, Jiangsu 215000 (CN)
(74) Representative: dompatent von Kreisler Selting Werner - Partnerschaft von Patent- und Rechtsanwälten mbB
(86) International application number: PCT/CN2022/143550
(87) International publication number: WO 2023/125842

(57) **Abstract**

The present invention provides an anti-UPAR single-domain antibody. Specifically, a plurality of anti-UPAR single-domain antibodies are obtained by means of phage display library screening technology. The anti-UPAR single-domain antibody of the present invention can efficiently bind to UPAR, and has application prospects in the treatment of related diseases.

## Description

### Technical field

The present invention relates to the field of biomedicine. Specifically, the present invention relates to an anti-UPAR single domain antibody.

### Background

In recent years, population aging has gradually become a focal point of social concern. Aging is directly linked to an increased incidence of infectious diseases, cardiovascular diseases, neurodegenerative diseases, autoimmune disorder diseases, and tumors, etc. One of the significant reasons for these diseases is organism aging accompanied with the disorder of immune function.

Several small-molecule anti-aging agents have been developed in recent years. They are able to eliminate senescent cells, while they have low therapeutic effects and significant side effects. Increasing researchers are dedicated to developing an "anti-aging agent" that can safely and effectively clear senescent cells. CAR-T therapy is a groundbreaking cancer treatment method at present. By using gene editing technology to equip T cells with a CAR (tumor chimeric antigen receptor) that is capable of targeting, it can achieve the purpose of identifying tumor cells, inducing immune responses, and killing tumor cells efficiently. This therapy has also provided new insights for researchers in the study of anti-aging agents. If CAR-T cells can identify senescent cells, it would transform the anti-tumor therapy into an anti-aging therapy. Traditional intervention methods in diseases focus on treatment after illness onset. With advances in medical technology, bringing the intervention point forward to prevent aging and aging-related malignancies will be a new trend.

Currently, representative anti-aging drugs include: rapamycin, dasatinib combined with quercetin, fisetin, UBX0101 and other anti-aging agents. However, these drugs have potential side effects. Firstly, their ability for targeted elimination of senescent cells is still not sufficiently precise, and will damage normal cells. Secondly, premature use of anti-aging drugs can lead to stem cell exhaustion, while delayed use affects their effectiveness. Thirdly, after a large number of senescent cells are killed, the remaining products are often not cleared in time, posing a potential health risk. Therefore, the development of a novel anti-aging drug is an unmet need, and the development of CAR-T cell drugs may become a trend in the treatment of aging. The first step in developing drugs that recognize senescent cells should be to identify the surface markers of senescent cells. A study has reported the analysis of transmembrane protein expression in senescent human and mouse cells based on RNA sequencing data from three independent aging models, ultimately identified the urokinase-type plasminogen activator receptor (UPAR) as the "identity card" of senescent cells.

However, in this field, there is currently a lack of nanobodies targeting UPAR that can be used to construct CAR-T cell drugs or other drugs targeting UPAR.

Therefore, there is a need in this field to develop an anti-UPAR nanobody.

### Summary of the invention

The purpose of the present invention is to provide a UPAR-targeting single domain antibody and use thereof.

In the first aspect of the present invention, it provides an anti-UPAR single domain antibody, wherein the complementarity determining regions (CDRs) of a VHH chain of the anti-UPAR single domain antibody is one or more selected from the group consisting of:
(1) CDR1 as shown in SEQ ID NO: 37, CDR2 as shown in SEQ ID NO: 53, and CDR3 as shown in SEQ ID NO: 54;
(2) CDR1 as shown in SEQ ID NO: 22, CDR2 as shown in SEQ ID NO: 23, and CDR3 as shown in SEQ ID NO: 24;
(3) CDR1 as shown in SEQ ID NO: 42, CDR2 as shown in SEQ ID NO: 43, and CDR3 as shown in SEQ ID NO: 44;
(4) CDR1 as shown in SEQ ID NO: 25, CDR2 as shown in SEQ ID NO: 26, and CDR3 as shown in SEQ ID NO: 27;
(5) CDR1 as shown in SEQ ID NO: 28, CDR2 as shown in SEQ ID NO: 29, and CDR3 as shown in SEQ ID NO: 30;
(6) CDR1 as shown in SEQ ID NO: 31, CDR2 as shown in SEQ ID NO: 32, and CDR3 as shown in SEQ ID NO: 33;
(7) CDR1 as shown in SEQ ID NO: 34, CDR2 as shown in SEQ ID NO: 35, and CDR3 as shown in SEQ ID NO: 36;
(8) CDR1 as shown in SEQ ID NO: 37, CDR2 as shown in SEQ ID NO: 38, and CDR3 as shown in SEQ ID NO: 39;
(9) CDR1 as shown in SEQ ID NO: 40, CDR2 as shown in SEQ ID NO: 26, and CDR3 as shown in SEQ ID NO: 41;
(10) CDR1 as shown in SEQ ID NO: 19, CDR2 as shown in SEQ ID NO: 20, and CDR3 as shown in SEQ ID NO: 21;
(11) CDR1 as shown in SEQ ID NO: 45, CDR2 as shown in SEQ ID NO: 46, and CDR3 as shown in SEQ ID NO: 47;
(12) CDR1 as shown in SEQ ID NO: 48, CDR2 as shown in SEQ ID NO: 49, and CDR3 as shown in SEQ ID NO: 50;
(13) CDR1 as shown in SEQ ID NO: 51, CDR2 as shown in SEQ ID NO: 38, and CDR3 as shown in SEQ ID NO: 52; and
(14) CDR1 as shown in SEQ ID NO: 16, CDR2 as shown in SEQ ID NO: 17, and CDR3 as shown in SEQ ID NO: 18.

In another preferred embodiment, the VHH chain of the anti-UPAR single domain antibody further comprises framework regions (FRs).

In another preferred embodiment, the FR has an amino acid sequence derived from any one of SEQ ID NOs: 1-15.

In another preferred embodiment, the amino acid sequence of the VHH chain of the anti-UPAR single domain antibody is selected from sequences shown in SEQ ID NOs: 1-15.

In another preferred embodiment, the amino acid sequence of the VHH chain of the anti-UPAR single domain antibody is selected from a sequence shown in SEQ ID NO: 3, 10 or 15.

In another preferred embodiment, the anti-UPAR single domain antibody is selected from the group consisting of: a monomer, a bivalent (bivalent antibody), a multivalent antibody, and a combination thereof.

In another preferred embodiment, the anti-UPAR single domain antibody is a bivalent.

In another preferred embodiment, the anti-UPAR single domain antibody is selected from the group consisting of: a humanized antibody, a camel-derived antibody, a chimeric antibody.

In the second aspect of the present invention, it provides an anti-UPAR antibody, which comprises one or more VHH chain(s) of the anti-UPAR single domain antibody according to the first aspect of the present invention.

In another preferred embodiment, the amino acid sequence of the VHH chain of the anti-UPAR single domain antibody is selected from sequences shown in SEQ ID NOs: 1-15.

In another preferred embodiment, the amino acid sequence of the VHH chain of the anti-UPAR single domain antibody is selected from a sequence shown in SEQ ID NO: 3, 10 or 15.

In another preferred embodiment, the anti-UPAR antibody is selected from the group consisting of: a monomer, a bivalent (bivalent antibody), a multivalent antibody, and a combination thereof.

In another preferred embodiment, the bivalent (or multivalent) refers that the amino acid sequence of the immunoconjugate contains two (or multiple) identical or different VHH chain sequences of the anti-UPAR single domain antibody according to the first aspect of the present invention.

In the third aspect of the present invention, it provides a polynucleotide encoding a protein selected from the group consisting of: the anti-UPAR single domain antibody of the first aspect of the present invention, or the antibody of the second aspect of the present invention.

In another preferred embodiment, the present invention relates to a nucleic acid molecule encoding the anti-UPAR single domain antibody of the present invention. The nucleic acid molecule of the present invention may be RNA, DNA or cDNA.

In the fourth aspect of the present invention, it provides an expression vector comprising the polynucleotide of the third aspect of the present invention.

In another preferred embodiment, the expression vector is selected from the group consisting of: DNA, RNA, a viral vector, a plasmid, a transposon, other gene transfer system, and a combination thereof.

In another preferred embodiment, the expression vector is a pcDNA3.4-hIgG1-Fc2 plasmid.

In the fifth aspect of the present invention, it provides a host cell comprising the expression vector of the fourth aspect of the present invention, or having the polynucleotide of the third aspect of the present invention integrated in its genome.

In another preferred embodiment, the host cell is selected from the group consisting of: a prokaryotic cell or a eukaryotic cell.

In another preferred embodiment, the host cell is selected from the group consisting of: *Escherichia coli*, a yeast cell, and a mammalian cell.

In another preferred embodiment, the host cell is a 293F cell.

In the sixth aspect of the present invention, it provides a method for producing an anti-UPAR single domain antibody, which comprises the steps of:
(a) culturing the host cell of the fifth aspect of the present invention under conditions suitable for producing a single domain antibody, thereby obtaining a culture containing the anti-UPAR single domain antibody;
(b) isolating and/or recovering the anti-UPAR single domain antibody from the culture; and
(c) optionally, purifying and/or modifying the anti-UPAR single domain antibody obtained in step (b).

In the seventh aspect of the present invention, it provides an immunoconjugate which comprises:
(a) the anti-UPAR single domain antibody of the first aspect of the present invention, or the anti-UPAR antibody of the second aspect of the present invention; and
(b) a coupling moiety selected from the group consisting of: a detectable label, a drug, a toxin, a cytokine, a radionuclide, an enzyme, a gold nanoparticle/nanorod, a nanomagnetic particle, a viral coat protein or VLP, and a combination thereof.

In another preferred embodiment, the radionuclide is selected from the group consisting of:
(i) a diagnostic isotope, which is selected from the group consisting of: Tc-99m, Ga-68, F-18, I-123, I-125, I-131, In-111, Ga-67, Cu-64, Zr-89, C-11, Lu-177, Re-188, and a combination thereof; and/or
(ii) a therapeutic isotope, which is selected from the group consisting of: Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, pd-103, P-32, K-42, Re-186, Re-188, Sm-153, Ra223, Ru-106, Na24, Sr89, Tb-149, Th-227, Xe-133 Yb-169, Yb-177, and a combination thereof.

In another preferred embodiment, the coupling moiety is a detectable label.

In another preferred embodiment, the coupling moiety is selected from the group consisting of: a fluorescent or luminescent label, a radioactive label, MRI (magnetic resonance imaging) or CT (electronic computer X-ray tomography technique) contrast agent, or an enzyme capable of producing a detectable product, a radionuclide, a biotoxin, a cytokine (such as IL-2, etc.), an antibody, an Fc fragment of an antibody, an scFv fragment of an antibody, a gold nanoparticle/nanorod, a viral particle, a liposome, a nanomagnetic particle, a prodrug activating enzyme (such as DT-diaphorase (DTD) or biphenyl hydrolase-like protein (BPHL)), or a nanoparticle in any form.

In another preferred embodiment, the immunoconjugate comprises: a multivalent (such as bivalent) VHH chains of the anti-UPAR single domain antibody according to the first aspect of the present invention.

In the eighth aspect of the present invention, it provides a use of the anti-UPAR single domain antibody of the first aspect of the present invention, the anti-UPAR antibody of the second aspect of the present invention, or the immunoconjugate of the seventh aspect of the present invention for preparing:
(1) a drug for preventing and/or treating a disease related to UPAR; and/or
(2) a reagent for detecting UPAR.

In another preferred embodiment, the disease related to UPAR is selected from the group consisting of: an aging-related disease, a cancer or tumor.

In another preferred embodiment, the cancer or tumor is selected from the group consisting of: hematological tumors, lymphomas, solid tumors, and a combination thereof.

In another preferred embodiment, the hematological tumor is selected from the group consisting of: acute myeloid leukemia (AML), multiple myeloma (MM), chronic lymphocytic leukemia (CLL), acute lymphocytic leukemia (ALL), diffuse large B cell lymphoma (DLBCL), and a combination thereof.

In another preferred embodiment, the lymphoma is selected from the group consisting of: Hodgkin lymphoma (HL), diffuse large B-cell lymphoma (DLBCL), follicular lymphoma (FL), chronic lymphocytic leukocyte (CLL), small lymphocytic lymphoma (SLL), marginal zone lymphoma (MZL), mantle cell lymphoma (MCL), Burkitt lymphoma (BL), and other complex B-cell non-Hodgkin lymphomas.

In another preferred embodiment, the solid tumor is selected from the group consisting of: gastric cancer, gastric cancer peritoneal metastasis, liver cancer, kidney tumor, lung cancer, small intestinal cancer, bone cancer, prostate cancer, colorectal cancer, breast cancer, colon cancer, cervical cancer, ovarian cancer, lymphoma, nasopharyngeal cancer, adrenal tumor, bladder tumor, non-small cell lung cancer (NSCLC), brain glioma, endometrial cancer, testicular cancer, colorectal cancer, urinary tract tumor, thyroid cancer, and a combination thereof.

In another preferred embodiment, the drug is used for treating an aging-related disease.

In another preferred embodiment, the aging-related disease is selected from the group consisting of: infectious disease, cardiovascular disease, neurodegenerative disease, autoimmune disease, tumor, and a combination thereof.

In another preferred embodiment, the drug is administered to a human or a non-human mammal.

In another preferred embodiment, the reagent is a diagnostic reagent, and preferably the diagnostic reagent is a detection slide or a detection plate.

In another preferred embodiment, the diagnostic reagent is used for: detecting UPAR or a fragment thereof in a sample.

In the ninth aspect of the present invention, it provides a pharmaceutical composition which comprises:
(i) the anti-UPAR single domain antibody of the first aspect of the present invention, the anti-UPAR antibody of the second aspect of the present invention, or the immunoconjugate of the seventh aspect of the present invention; and
(ii) a pharmaceutically acceptable carrier.

In the tenth aspect of the present invention, it provides a recombinant protein which comprises:
(i) the anti-UPAR single domain antibody of the first aspect of the present invention, or the anti-UPAR antibody of the second aspect of the present invention; and
(ii) optionally, a tag sequence to assist expression and/or purification.

In another preferred embodiment, the tag sequence is selected from: an Fc tag, an HA tag and a His tag.

In another preferred embodiment, the recombinant protein specifically binds to UPAR.

In the eleventh aspect of the present invention, it provides a kit which comprises the anti-UPAR single domain antibody of the first aspect of the present invention, the anti-UPAR antibody of the second aspect of the present invention, or the immunoconjugate of the seventh aspect of the present invention.

In the twelfth aspect of the present invention, it provides a method of preventing and/or treating a disease related to UPAR, which comprises a step of administering the anti-UPAR single domain antibody of the first aspect of the present invention, the anti-UPAR antibody of the second aspect of the present invention, or the immunoconjugate of the seventh aspect of the present invention, to a subject in need thereof.

In another preferred embodiment, the subject is selected from mammal, such as a human.

In another preferred embodiment, the disease related to UPAR is selected from cancers and autoimmune diseases.

In the thirteenth aspect of the present invention, it provides a method for detection of UPAR or a fragment thereof in a sample *in vitro,* wherein the method comprises the steps of:
(1) contacting the sample with the anti-UPAR single domain antibody of the first aspect of the present invention, the anti-UPAR antibody of the second aspect of the present invention, or the immunoconjugate of the seventh aspect of the present invention *in vitro*; and
(2) detecting whether an antigen-antibody complex is formed, wherein the formation of the complex indicates the presence of UPAR or a fragment thereof in the sample.

In another preferred embodiment, the detection includes a diagnostic detection or a non-diagnostic detection.

In the fourteenth aspect of the present invention, it provides a method for diagnosis of a disease related to UPAR, which comprises the steps of:
(i) collecting a sample from a subject to be diagnosed, contacting the sample with the anti-UPAR single domain antibody of the first aspect of the present invention, the anti-UPAR antibody of the second aspect of the present invention, or the immunoconjugate of the seventh aspect of the present invention; and
(ii) detecting whether an antigen-antibody complex is formed, wherein the formation of the complex indicates that the subject suffers from a disease related to UPAR.

In the fifteenth aspect of the present invention, it provides a method for preparing a recombinant polypeptide which is the anti-UPAR single domain antibody of the first aspect of the present invention or the anti-UPAR antibody of the second aspect of the present invention, wherein the method comprises the steps of:
(a) culturing the host cell according to the fifth aspect of the present invention under conditions suitable for expression; and
(b) isolating the recombinant polypeptide from the culture.

It should be understood that, within the scope of the present invention, the technical features specifically described above and below (such as the Examples) can be combined with each other, thereby constituting a new or preferred technical solution which will not be described herein one by one due to space limitations.

### DESCRIPTION OF DRAWINGS

The following drawings are used to illustrate specific embodiments of the present invention and are not intended to limit the scope of the present invention as defined by the claims.
Fig. 1 shows the results of UPAR-His recombinant protein expression detected by SDS-PAGE. Lane 1 represents the protein band of UPAR-His, and lane M represents the protein Marker.
Fig. 2 shows the activity detection results of UPAR-His recombinant protein.
Fig. 3 shows the ELISA detection results of immune serum.
Fig. 4 shows the PCR amplification results of VHH fragments. The left image shows the results of the first round PCR, and the right image shows the results of the second round PCR.
Fig. 5 shows the alignment of sequencing results of different mono clones.
Fig. 6 shows the results of solid-phase panning of antibody phages detected by Elisa.
Figs. 7A-7D show the flow cytometry detection results of different clonal antibodies obtained through phage display library panning.
Figs. 8A-8C show the flow cytometry detection results after expression and purification of UPAR antibodies from different clones.
Fig. 9 presents the affinity detection results of three representative UPAR antibodies (1-F09, 3-E04, 3-H02).

### DETAILED DESCRIPTION

After extensive and intensive research, the present inventors have successfully obtained multiple anti-UPAR single-domain antibodies through large amount of screening. Specifically, the present invention used UPAR-Fc antigen protein to immunize llamas, screened the immune single domain antibody gene library (phage display library) using phage display technology, and performed panning and identification to obtain single domain antibody genes targeting UPAR. Experimental results indicate that the anti-UPAR single-domain antibodies obtained by the present invention can effectively bind to UPAR, and has application prospects in the treatment of UPAR-related diseases, specifically tumors and aging-related diseases. On this basis, the present invention has been completed.

### Terms

As used herein, the terms "antibody according to the present invention", "antibody of the present invention", "anti-UPAR single domain antibody of the present invention" and "anti-UPAR single domain antibody" have the same meaning and can be used interchangeably to refer to a single domain antibody that specifically recognizes and binds to UPAR.

### Antibody

The term "antibody" or "immunoglobulin" as used herein refers to a heterotetrameric glycoprotein having the same structural feature of about 150,000 daltons consisting of two identical light chains (L) and two identical heavy chains (H). Each light chain is linked to a heavy chain by a covalent disulfide bond, and the numbers of disulfide bonds between the heavy chains of different immunoglobulin isoforms are different. Each heavy and light chain also has regularly spaced intrachain disulfide bonds. One end of each heavy chain has a variable region (VH) followed by a plurality of constant regions. There is a variable region (VL) at one end of each chain and a constant region at the other end; the constant region of the light chain corresponds to the first constant region of the heavy chain; the variable region of the light chain corresponds to the variable region of the heavy chain. An interface is formed between the variable regions of the light and heavy chains by particular amino acid residues.

As used herein, the terms "single domain antibody", "VHH", "nanobody", "single domain antibody (sdAb, or nanobody)" have the same meaning and can be used interchangeably to refer to the heavy chain variable region of an antibody clone constructing a single domain antibody (VHH) composed of only one heavy chain variable region, which is the smallest antigen-binding fragment with complete function. Usually, the antibody with natural deletion of light chain and heavy chain constant region 1(CH1) is obtained first, and then the variable region of the antibody heavy chain is cloned to construct a single domain antibody (VHH) composed of only one heavy chain variable region.

As used herein, the term "variable" means that some certain portions of the variable region of an antibody differ in sequence and contribute to the binding and specificity of each particular antibody to its particular antigen. However, the variability is not evenly distributed throughout the antibody variable region. It is concentrated in three regions in the light and heavy chain variable regions called complementarity determining regions (CDRs) or hypervariable regions. The more conserved portions of the variable regions are referred as framework regions (FRs). The variable regions of the natural heavy and light chains each comprises four FR regions, which are in a substantially β-sheet configuration, and are linked by three CDRs that form the linker ring and, in some cases, form a partial β-sheet structure. The CDRs in each chain stand close together through FR regions and form the antigen-binding site of the antibody together with the CDRs of the other chain (see Kabat et al., NIH Publ. No. 91-3242, Vol. I, 647-669 (1991)). Constant regions are not directly involved in the binding of the antibodies to the antigens, but they exhibit different effector functions, such as antibody-dependent cellular cytotoxicity involved in antibodies.

As known to those skilled in the art, an immunoconjugate and the fusion expression product includes: a conjugate formed by a drug, a toxin, a cytokine, a radionuclide, an enzyme and other diagnostic or therapeutic molecules connecting to the antibody or a fragment thereof. The present invention also comprises a cell surface marker or antigen binding to the nanobody against UPAR or a fragment thereof.

As used herein, the terms "heavy chain variable region" and "VH" can be used interchangeably.

As used herein, the terms "variable region" and "complementarity determine region (CDR)" can be used interchangeably.

In a preferred embodiment of the present invention, the heavy chain variable region of the antibody comprises three complementarity determining regions, CDR1, CDR2, and CDR3.

In a preferred embodiment of the present invention, the heavy chain of the antibody comprises the above-mentioned heavy chain variable region and a heavy chain constant region.

In the present invention, the terms "the antibody of the present invention", "the protein of the present invention", or "the polypeptide of the present invention" can be used interchangeably and all refer to an polypeptide specifically binding to UPAR, e.g., a protein or polypeptide with a heavy chain variable region. They can contain or do not contain starting methionine.

The invention also provides other proteins or fusion expression products comprising the antibody of the present invention. Specifically, the present invention includes any protein or protein conjugate and fusion expression product (i.e., immunoconjugate and fusion expression product) having a heavy chain containing variable regions, as long as the variable region is the same as or has at least 90% homology, preferably at least 95% homology with the variable region of the heavy chain of the antibody of the present invention.

The terms "specific binding", "selective binding", "selectively bind", and "specifically bind", refer to the binding of an antibody to an epitope on a predetermined antigen. Usually, an antibody has an affinity (KD) that is approximately less than 10⁻⁷M, such as approximately less than 10⁻⁹M, 10⁻⁹M or 10⁻¹⁰M or less.

In general, the antigen-binding properties of an antibody can be described by three specific regions located in the heavy chain variable region, referring as variable regions (CDRs), and separated into four framework regions (FRs). The sequences of four FRs amino acids are relatively conservative and do not directly participate in the binding reaction. A cyclic structure is formed by these CDRs which are close to each other in the spatial structure by the β-sheets formed by the FRs between them, and the CDRs on the heavy chains and the CDRs on the corresponding light chains constitute the antigen-binding sites of the antibody. The amino acid sequence of the same type of antibody can be used to determine which amino acids have constituted the FR or CDR regions.

The variable regions of the heavy chain of the antibody of the present invention are of particular interest because at least part of them involves binding antigens. Therefore, the present invention includes molecules with heavy chain variable regions of antibodies with CDRs, as long as their CDRs have more than 90% (preferably more than 95%, most preferably more than 98%) homology with the CDRs identified here.

The present invention includes not only intact antibodies, but also immunologically active fragments of antibody fragments or fusion proteins formed by antibodies and other sequences. Therefore, the present invention also includes fragments, derivatives and analogs of the antibodies.

As used herein, the terms "fragment", "derivative" and "analog" refer to a polypeptide basically maintaining the same biological function or activity of the antibody of the present invention. The polypeptide fragment, derivative or analog of the present invention may be (i) a polypeptide with one or more conservative or non-conservative amino acid residues (preferably the conservative amino acid residues) being substituted, while such substituted amino acid residues may or may not be encoded by genetic code, or (ii) a polypeptide having substituted group(s) in one or more amino acid residues, or (iii) a polypeptide formed by fusion of the matured polypeptide with another compound (such as the compound that prolongs the half-life of the polypeptide, such as polyethylene glycol), or (iv) a polypeptide formed with additional amino acid sequence fused to said polypeptide sequence (such as, leader sequence, secretion sequence, or a sequence or a protein sequence used to purify the polypeptide, or a fusion protein formed with 6His tag). According to the teaching of the present application, these fragments, derivatives, and analogs are within the scope commonly known by those skilled in the art.

The antibody of the present invention refers to a polypeptide having UPAR binding activity and comprising the above-mentioned CDR regions. The term also includes variant forms of polypeptides comprising the CDR regions described above that have the same function as the antibody of the present invention. These variant forms include, but are not limited to, deletion insertion and/or substitution of one or more amino acids (typically 1-50, preferably 1-30, more preferably 1-20, most preferably 1-10), and addition of one or several amino acids (typically at most 20, preferably at most 10, more preferably at most 5) at the C-terminus and/or N-terminus. For example, in the art, the protein's functions are usually unchanged when an amino acids is substituted by a similar or analogous one. Also, for example, the addition of one or several amino acids at the C-terminus and/or the N-terminus will not normally alter the function of the protein. The term also includes active fragments and active derivatives of the antibody of the present invention.

The variant forms of the antibody include homologous sequences, conserved variants, allelic variants, natural mutants, induced mutants, proteins encoded by a DNA capable of hybridizing to the coding DNA of the antibody of the present invention under high or low stringency conditions, and a polypeptide or protein obtained using an antiserum against the antibody of the present invention.

The present invention also provides other polypeptides, such as fusion proteins containing antibodies or fragments thereof. In addition to the almost full-length polypeptide, the present invention also includes fragments of the human antibody of the present invention. Typically, the fragment has at least about 50 contiguous amino acids, preferably at least about 50 contiguous amino acids, more preferably at least about 80 contiguous amino acids, and most preferably at least about 100 contiguous amino acids of the antibody of the present invention.

In the present invention, "the conservative variant of the antibody of the present invention" refers to a polypeptide that comprises at most 10, preferably at most 8, more preferably at most 5, most preferably at most 3 amino acids replaced by amino acids with the same or similar properties compared with the amino acid sequence of the antibody of the present invention. These conservatively variant polypeptides are preferably produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gin (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The invention also provides a polynucleotide molecule encoding the antibody or a fragment thereof or a fusion protein thereof. The polynucleotides of the present invention can be in a form of DNA or RNA. DNA forms include cDNA, genomic DNA, or synthetic DNA. DNA can be single-stranded or double-stranded. DNA can be the coding strand or the non-coding strand.

The polynucleotides encoding the mature polypeptides of the present invention comprise coding sequences encoding only the mature polypeptide; coding sequences of the mature polypeptide and various additional coding sequences; coding sequences (and optionally additional coding sequences) of the mature polypeptide, and non-coding sequences.

The term "polynucleotide encoding a polypeptide" may include a polynucleotide that encodes the polypeptide, or a polynucleotide that also includes additional coding and/or non-coding sequences.

The present invention also relates to polynucleotides that hybridize to the sequences as described above and having at least 50%, preferably at least 70%, more preferably at least 80% identical between the two sequences. In particular, the present invention relates to polynucleotides that can hybridize to the polynucleotides of the present invention under stringent conditions. In the present invention, "stringent conditions" means: (1) hybridization and elution at lower ionic strength and higher temperature, such as 0.2 × SSC, 0.1% SDS, 60 °C; or (2) hybridization adding a denaturant, such as 50% (v/v) formamide, 0.1% calf serum/0.1% Ficoll, 42 °C, or the like; or (3) hybridization only occurs when the identity between the two sequences is at least 90%, more preferably 95% or more. And the polypeptide encoded by the hybridizable polynucleotide has the same biological function and activity as the mature polypeptide.

The whole length of the nucleotide sequence or the fragment thereof of the antibody of the present invention can be obtained via PCR amplification, recombinant method or artificial synthesis. One feasible method is to synthesize relevant sequences by artificial method, especially when the fragment is short in length. Usually, several small fragments are synthesized first, and then are linked together to obtain a fragment with a long sequence. In addition, the sequence coding the heavy chain and the expression tag (e.g. 6His) can be fused together to form a fusion protein.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods. The biomolecules (nucleic acids, proteins, etc.) involved in the present invention include biomolecules that exist in an isolated form.

At present, DNA sequences encoding the protein of the invention (or fragments thereof, or derivatives thereof) can be completely obtained by chemical synthesis. The DNA sequence can then be introduced into a variety of existing DNA molecules (or vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Representative examples are: *Escherichia coli*, *streptomactinus*, bacterial cells of *salmonella typhimurium*; fungal cells such as yeast; insect cells of Drosophila S2 or SF9; animal cells of CHO, COS7, 293 cells, etc.

Transformation of a host cell with a recombinant DNA can be carried out by conventional techniques well known to those skilled in the art. When the host is a prokaryotic organism such as *Escherichia coli*, competent cells that can absorb DNA can be harvested after the exponential growth phase then treated with the CaCl₂ method, and the steps used are well known in the art. Another method is to use MgCl₂. If necessary, the transformation can also be carried out by electroporation. When the host is a eukaryote, the following DNA transfection methods can be selected: calcium phosphate co-precipitation method, conventional mechanical methods such as microinjection, electroporation, liposome packaging, etc.

The obtained transformants can be cultured by conventional methods to express the polypeptide encoded by the gene of the present invention. Depending on the host cell used, the medium used during the culture can be selected from various conventional mediums. The culture is carried out under conditions suitable for the growth of the host cell. When the host cell has grown to an appropriate cell density, a suitable method (such as temperature conversion or chemical induction) is used to induce the selected promoter, and the cell is cultured for another period of time.

The recombinant polypeptide described in the above method can be expressed intracellularly or on the cell membrane, or be secreted out of the cell. If desired, recombinant proteins can be isolated and purified by various separation methods utilizing their physical, chemical, and other properties. These methods are well known to those skilled in the art. Examples of such methods include, but are not limited to, conventional renaturation treatments, treatment with a protein precipitant (salting-out method), centrifugation, osmosis cell disruption, super-treatment, ultracentrifugation, molecular sieve chromatography (gel filtration), adsorption chromatography, ion-exchange chromatography, high performance liquid chromatography (HPLC) and various other liquid chromatography techniques and combinations of these methods.

The antibody of the present invention can be used alone, or can be combined or coupled with a detectable label (for diagnostic purposes), a therapeutic agent, a PK (protein kinase) modified moiety, or any combination of these substances.

Detectable labels for diagnostic purposes include, but are not limited to: fluorescent or luminescent labels, radioactive labels, MRI (magnetic resonance imaging) or CT (electronic computer X-ray tomography technique) contrast agents, or enzymes capable of producing detectable products.

A therapeutic agent that can be combined or coupled with the antibody of the present invention includes, but is not limited: 1. a radionuclide; 2. a biological toxin; 3. a cytokine such as IL-2, etc; 4. a gold nanoparticle/nanorod; 5. a viral particle; 6. a liposome; 7. a magnetic nanoparticle; 8. a prodrug-activating enzyme (e. g., DT-diaphorase (DTD) or biphenyl hydrolase-like protein (BPHL)).

### UPAR

As used herein, the term "UPAR" refers to urokinase-type plasminogen activator receptor. UPAR is a multi-domain glycoprotein cell surface receptor anchored to the cell membrane via GPI, which can bind urokinase-type plasminogen activator (uPA) with high affinity, thereby promoting the activation of plasminogen cells. UPAR can promote the degradation of extracellular matrix in the process of fibrinolysis, wound healing, or tumorigenesis, and can promote tumor cell motility, invasion, and survival. After ligand binding, a part of UPAR is proteolytically cleaved to generate soluble UPAR (sUPAR).

Examination of previously published data on protein and RNA expression in human tissues shows that UPAR is either undetected or present at low levels in most human organs including the central nervous system, heart, and liver. However, studies have found that UPAR is highly expressed in both *in vitro* and *in vivo* senescent cells. It has been confirmed in various models *in vitro* and *in vivo* that senescent cells can induce the expression of UPAR. An increase in the number of UPAR-positive cells and an increase in the level of sUPAR in serum can be observed in each model system. These results all indicate that UPAR is an excellent target for CAR-T cells targeting senescent cells. In addition, UPAR is also expressed in many solid tumor cell lines, indicating that UPAR is also an ideal target for targeting solid tumors.

### Pharmaceutical composition

The present invention further provides a composition. Preferably, the composition is a pharmaceutical composition comprising the antibody, or an active fragment or a fusion protein thereof, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated. The formulated pharmaceutical composition may be administered by conventional routes, including (but not limited to): intraperitoneal, intravenous, or topical administration.

The pharmaceutical composition according to the present invention comprises a safe and effective amount (e.g., 0.001-99 wt %, preferably 0.01-90 wt %, more preferably 0.1-80 wt %) of the antibody according to the present invention (or a conjugate thereof) and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The administration amount of an active ingredient is a therapeutically effective amount, for example, about 10 µg per kilogram of body weight to about 50 mg per kilogram of body weight daily. In addition, the polypeptide according to the present invention may also be used in combination with an additional therapeutic agent.

When a pharmaceutical composition is used, a safe and effective amount of immunoconjugate is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 10 mg per kilogram of body weight. Of course, a specific amount should also depend on the factors such as administration route and physical conditions of a patient, which fall into the skills of skilled physicians.

### Anti-UPAR single domain antibody

The present invention provides an anti-UPAR single domain antibody which is capable of specifically binding to UPAR.

In one aspect of the present invention, it provides an anti-UPAR single domain antibody, wherein complementarity determining regions (CDRs) of the VHH chain of the anti-UPAR single domain antibody is one or more selected from the group consisting of:
(1) CDR1 as shown in SEQ ID NO: 37, CDR2 as shown in SEQ ID NO: 53, and CDR3 as shown in SEQ ID NO: 54;
(2) CDR1 as shown in SEQ ID NO: 22, CDR2 as shown in SEQ ID NO: 23, and CDR3 as shown in SEQ ID NO: 24;
(3) CDR1 as shown in SEQ ID NO: 42, CDR2 as shown in SEQ ID NO: 43, and CDR3 as shown in SEQ ID NO: 44;
(4) CDR1 as shown in SEQ ID NO: 25, CDR2 as shown in SEQ ID NO: 26, and CDR3 as shown in SEQ ID NO: 27;
(5) CDR1 as shown in SEQ ID NO: 28, CDR2 as shown in SEQ ID NO: 29, and CDR3 as shown in SEQ ID NO: 30;
(6) CDR1 as shown in SEQ ID NO: 31, CDR2 as shown in SEQ ID NO: 32, and CDR3 as shown in SEQ ID NO: 33;
(7) CDR1 as shown in SEQ ID NO: 34, CDR2 as shown in SEQ ID NO: 35, and CDR3 as shown in SEQ ID NO: 36;
(8) CDR1 as shown in SEQ ID NO: 37, CDR2 as shown in SEQ ID NO: 38, and CDR3 as shown in SEQ ID NO: 39;
(9) CDR1 as shown in SEQ ID NO: 40, CDR2 as shown in SEQ ID NO: 26, and CDR3 as shown in SEQ ID NO: 41;
(10) CDR1 as shown in SEQ ID NO: 19, CDR2 as shown in SEQ ID NO: 20, and CDR3 as shown in SEQ ID NO: 21;
(11) CDR1 as shown in SEQ ID NO: 45, CDR2 as shown in SEQ ID NO: 46, and CDR3 as shown in SEQ ID NO: 47;
(12) CDR1 as shown in SEQ ID NO: 48, CDR2 as shown in SEQ ID NO: 49, and CDR3 as shown in SEQ ID NO: 50;
(13) CDR1 as shown in SEQ ID NO: 51, CDR2 as shown in SEQ ID NO: 38, and CDR3 as shown in SEQ ID NO: 52; and
(14) CDR1 as shown in SEQ ID NO: 16, CDR2 as shown in SEQ ID NO: 17, and CDR3 as shown in SEQ ID NO: 18.

In another preferred embodiment, the amino acid sequence of the VHH chain of the anti-UPAR single domain antibody is selected from sequences shown in SEQ ID NOs: 1-15. More preferabley, the VHH chain of the anti-UPAR single domain antibody has an amino acid sequence shownin SEQ ID NO: 3, 10 or 15.

The nanobody of the present invention has a high affinity for UPAR. The high affinity refers that the antibody of the present invention binds to UPAR with an affinity (KD) of lower than 10⁻⁶ M, preferably lower than 10⁻⁷ M, more preferably lower than 10⁻⁸ M or less.

The nanoantibody of the present invention have the advantages of nanoantibodies, such as small molecular weight, fast tissue penetration, high solubility and stability, high antigen binding specificity and low immunogenicity. Moreover, due to the small molecular weight of the nanoantibody drug, the nanoantibody can also recognize some invisible antigenic epitopes that cannot be recognized by the monoclonal antibody drug. In addition, in the human body, the nanoantibody may produce lower immunogenicity than the murine antibody. Thus, antibody drugs or CAR-T drugs constructed with a nanobody sequence have greater advantages than antibody drugs or CAR-T drugs constructed with a murine single-chain antibody sequence.

The UPAR nanoantibody of the present invention can be used to construct UPAR nanoantibody drugs or CAR-T drugs based on UPAR nanoantibody sequences for targeting treatment of aging-related diseases or tumors.

In another aspect of the present invention, it provides an anti-UPAR antibody. The antibody may be monovalent or multivalent and may comprise one or more identical or different VHH chains of the anti-UPAR single domain antibody of the present invention. Preferably, the VHH chain have an amino acid sequence shown in SEQ ID NO: 3, 10 or 15.

### Detection Method

The present invention also relates to a method for detecting UPAR. The steps of the method are roughly as follows: obtaining a cell and/or tissue sample; dissolving the sample in a medium; and detecting the level of UPAR in the dissolved sample.

In the detection method of the present invention, the sample used is not particularly limited, and a representative example is a cell-containing sample present in a cell preservation solution.

### Kit

The present invention also provides a kit containing the antibody (or a fragment thereof) or the detection plate of the present invention. In a preferred embodiment of the present invention, the kit further comprises a container, an instruction, and a buffer, etc.

The present invention also provides a detection kit for detecting the UPAR level, which comprises an antibody that recognizes UPAR proteins, a lysis medium for dissolving a sample, common reagents and buffers required for detection, such as various buffers, detection labels, detection substrates, etc. The detection kit may be an *in vitro* diagnostic device.

### Application

As described above, the antibody of the present invention has a wide range of biological application value and clinical application value, and its application relates to the diagnosis and treatment, basic medical research, biological research and other fields of the UPAR related diseases. One preferred application is for clinical diagnosis, prevention and treatment for UPAR related diseases.

### Main advantages of the present invention include:

1) The antibody of the present invention can specifically bind to UPAR;
2) The antibody of the present invention has a high affinity for UPAR;

The UPAR nanobody of the present invention has the advantages of small molecular weight, fast tissue penetration, high solubility and stability, high antigen binding specificity, low immunogenicity and the like. The UPAR nanobody of the present invention can be used in development of drugs for anti-aging or cancer treatment.

The invention is further illustrated below in conjunction with specific embodiments. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

### Experimental Reagents, Consumables and Equipment:

Main reagents: Agar (Sigma, CAT# A1296); Peptone (Sigma, CAT#93926); Yeast Extract (OXOID, CAT#: LP0021); Sodium Chloride (Aladdin, CAT#: C111533); Potassium Chloride (Aladdin, CAT#: P112133); Magnesium Sulfate (Sinopharm, CAT#: 10013018); Magnesium Chloride (Sinopharm, CAT#: 10012818); Glucose (Sangon, CAT#: GT1991); SfiI (NEB, CAT#: R0123L); T4 DNA Ligase (TaKaRa, CAT#: 2011A); PrimeScript^{™} II 1st Strand cDNA Synthesis Kit (TaKaRa, CAT#: 6210B); NuHi power Mix (NUHIGH Bio, CAT#: NH9303); 3M Sodium Acetate (pH 5.2-6) (Sigma, CAT#: 126-96-5); DNA Fragment Recovery Kit (TakaRa, CAT#: 9761); Gel Recovery Kit (Qiagen, CAT#: 28706); Tiangen Plasmid Extraction Kit (Tiangen, CAT#: DP117); HRP-M13 (Sino Biological, CAT#: 11973-MM05); PE-anti-Human IgG (eBioscience, Cat#: 12-4998-82); Rabbit anti-Llama IgG (H+L) Secondary Antibody [HRP] (Novus, CAT# NBP1-75095); SS320 Competent Cells (iCarTab); pComF Phage Display Vector (iCarTab); NHS-biotin (APExBIO, CAT#: A8002); HRP-Streptavidin (Boster, CAT#: BA1088); Streptavidin magnetic beads (NEB, CAT#:S14205); Antibody affinity detection buffer: HBS-EP+ 10X(GE, Cat# BR100669); Amino coupling kit(GE, Cat#BR100050); 10mM Glycine 2.5(GE, Cat#BR100356); S Series CM5 chip(GE,Cat#29149603); PBS (Gbico, CAT# 14190-250); DMEM (Gbico, CAT# 41965-062); RPMI1640 (Gbico, CAT# 61870044); FBS (Gbico, CAT# 10099-141); Genomic DNA Purification Kit (Lifetech, CAT#K0512); Polybrene(Sigma, CAT#107689-10G); LVtransm transfection reagent(iCarTab, Cat# LVTran100); Lymphocyte separation medium (Stem Cell, CAT# 18051); X-Vivo 15 serum-free medium(Lonza, CAT#04-418Q); Dynabeads^{®} Human T-Expander CD3/CD28(Thermo, CAT# 11141D); IL-2 (Beijing Yuance Pharmaceutical Co., Ltd., National Drug Approval No. S10980067); IL-7(PrimeGene, CAT#101-07); IL-15(PrimeGene, CAT#101-15); Bright-GloTM Luciferase Assay System(Promega, CAT#E2610); IL-2 ELISA kit (R&D, CAT#D2050); IFN-γ ELISA kit (R&D, CAT# DIF50).

Main consumables: 50mL Falcon Tubes (Corning, CAT#352070); Electroporation Cuvettes (Bio-Rad 0.2cm); RNase-free 1.5ml EP Tubes (QSP, CAT#: 509-GRD-Q); 200 µL RNase-free PCR Tubes (Axygen, PCR-02D-C); T125 Flask (Corning, CAT# 431143); 15mL Falcon centrifuge tubes (Corning, CAT# 430052); 6-well plates (Corning, CAT# 3516); 96-well plates (Corning, CAT# 3365).

Main equipments: Electroporator (Eppendorf Multiporator); Centrifuge (Xiangyi H1650R); Constant Temperature Incubator (Shanghai Jinghong DNP-9052); Constant Temperature Shaking Incubator (Langyue DZ-85A); Super Clean Bench (Sujing Antai SW-CJ-1FD); PCR (Applied Biosystems ABI2720); Biological Safety Cabinet (Haier HR40-IIA2); Flow Cytometer (Thermo Attune Nxt flow cytometer); Thermo 3111 CO2 incubator; BiaCore T200.

### Experimental Methods

### 1. Preparation of Antigen

1) Sequence of the extracellular domain of UPAR (residues 23-303) was generated by gene synthesis, followed by attachment of a His tag at the C-terminus. The construct was subcloned into a eukaryotic expression vector to form the antigen expression vector.
2) The prepared UPAR-His protein expression vectors were subjected to large-scale plasmid extraction, then transfected into 293 cells. Cells were continuously cultured for 8 days and centrifugated to collect the supernatant. The supernatant was filtered through a 0.45 µm filter membrane, and the filtrate was transferred to a sterile centrifuge tube. Purification was performed using a Ni-NTA column.
3) Biological activity of the purified antibodies was detected using uPA.

### 2. Antigen Immunization of Llama

The above prepared antigen was used to immunize a llama through subcutaneous multi-point injection for a total of three times. The schedule is shown in the table below.

**Table 1 Llama Immunization Schedule**

| | |
|---|---|
| Primary immunization | 5 mL blood was collected as negative serum before immunization; adjuvant was mixed with antigen (1mg) at 1:1, emulsified, and subcutaneously injected at multiple points. |
| Second immunization | Antigen (0.5 mg) was mixed with adjuvant at 1:1, emulsified, and subcutaneously injected at multiple points. |
| Third immunization | Antigen (0.5 mg) was mixed with adjuvant at 1:1, emulsified, and subcutaneously injected at multiple points. |
| | 100 mL peripheral blood was sampled 10 days after the third immunization for construction of phage display library. |

### 3. Detection of Immunological Potency

1) 5ml of peripheral blood was collected, and the centrifuge tube containing the blood sample was placed in an incubator at 37 °C for 1 hour. Afterward, the blood sample was transferred to 4 °C overnight.
2) The serum was transferred to a new sterile centrifuge tube and centrifuged at 5000 rpm for 20 minutes. The immunological potency was then detected by ELISA.

### 4. PBMC Isolation

100ml of peripheral blood was collected, and PBMCs were isolated using lymphocyte separation medium.

RNA was extracted and subjected to reverse transcription using the PrimeScript^{™} II 1st Strand cDNA Synthesis Kit to prepare cDNA. The method was as follows:
Reaction mixture MIX1 shown in Table 2 was prepared in a 200 µL PCR tube:

**Table 2 Formula of reaction mixture MIX1**

| Reagent | Quantity |
|---|---|
| Oligo dT Primer (50 µM) | 8µL |
| dNTP Mixture (10 mM each) | 8µL |
| Total RNA sample | 20µg |
| RNase-Free Water | Up to 80 µL |

After incubation at 65 °C for 5 minutes, the mixture was rapidly cooled on ice.

The reaction solution shown in Table 3 was prepared in the same PCR tube as described above:

**Table 3 Formula of reaction solution**

| Reagent | Quantity |
|---|---|
| The above denatured reaction mixture | 80 µL |
| 5×PrimeScript II Buffer | 32µL |
| RNase Inhibitor (40 U/µL) | 4µL |
| PrimeScript II RTase (200 U/µL) | 8µL |
| RNase-Free Water | 36µL |

After thorough mixing, 80 µL of the solution was dispensed into each tube and placed in a PCR instrument at 42 °C for 1 hour, followed by heat-inactivation at 70 °C for 15 minutes. Finally, the cDNA samples were stored on ice or at -20°C for long-term preservation.

### 5. Amplification of VHH fragments

The reaction system of the first round PCR was prepared according to Table 4 (50 µL/tube).

**Table 4 Reaction system of first round PCR**

| Component | Quantity |
|---|---|
| Forward primer (5 µM) | 2 |
| Reverse primer (10 µM) | 1 |
| NuHi Power mix (2×) | 25 |
| cDNA template | 2 |
| Sterile water | 20 |

After the preparation of PCR reaction system, the PCR instrument was set up according to the procedure shown in Table 5:

**Table 5 PCR program**

| Program | Temperature | Time | Number of cycles |
|---|---|---|---|
| Pre-denaturation | 95°C | 10 min | |
| Denaturation | 95°C | 15S | 30X |
| Annealing | 55°C | 30S | |
| Extension | 68°C | 1 min | |
| Final extension | 68°C | 10 min | |

Electrophoresis analysis on PCR products was performed by using 1% agarose, and fragments with a molecular weight of approximately 750 bp were isolated. PCR products were recovered by a gel recovery kit and subjected to the determination of concentration by NanoDrop.

The reaction system of the second round PCR was prepared according to Table 6 (50 µL/tube).

**Table 6 Reaction system of second round PCR**

| Component | Quantity |
|---|---|
| 2^{nd} F primer | 2 |
| 2^{nd} R primer | 2 |
| NuHi Power mix (2×) | 25 |
| Products recovered from the first round PCR | 200 ng |
| Sterile water | Add to 50 µL |

After the preparation of PCR reaction system, the PCR instrument was set up according to the procedure in Table 7:

**Table 7 Second round PCR program**

| Program | Temperature | Time | Number of cycles |
|---|---|---|---|
| Pre-denaturation | 95°C | 10 min | |
| Denaturation | 95°C | 15S | 25X |
| Annealing | 55°C | 30S | |
| Extension | 68°C | 1min | |
| Final extension | 68°C | 10 min | |

Electrophoresis analysis on PCR products was performed by using 1% agarose, and VHH fragments with a molecular weight of approximately 400 bp were isolated. PCR products of VHH were recovered by a gel recovery kit and subjected to the determination of concentration by NanoDrop.

### 6. Construction of Phage Display Library

Construction of Phage Display Vector:
1) The pCom F vector and the previously obtained recovered PCR gel product of VHH were digested with SfiI at 50 °C overnight, respectively.
2) The pCom F vector fragment was isolated using 1% agarose gel. The gel of 5000bp vector fragment was excised and recovered. Meanwhile, the PCR-digested product was purified using DNA Fragment Recovery Kit, and subjected to the determination of concentration by using NanoDrop.
3) The digested pCom F vector and VHH fragment were ligated using T4 ligase at 16 °C overnight.

Electrotransformation of phage ligation product into *E. coli*:
1) Electroporation cuvettes, ligation product, and electrotransformation competent cells were placed on ice for pre-cooling;
2) The pre-cooled library ligation product was added to the electrotransformation competent cells, kept on ice for 1 minute, and 70 µL of DNA/competent cell mixture was added to each electroporation cuvette, which was then placed on ice;
3) Electrotransformation was performed at 2500V for 5ms;
4) After electroporation, cells were immediately resuspended by adding SOC culture medium equilibrated to room temperature, and cultured in a shaker at 37 °C for 1 hour.
5) 15mL of the bacterial suspension was directly used for phage rescue, while the remaining 5mL of the electroporation product was mixed with an equal volume of 50% glycerol, thoroughly mixed, and stored at -80 °C.
6) Additionally, 20 µL of the bacterial suspension was diluted in 980 µL of 2YT culture medium, and 100 µL of the diluted product was further diluted in 900 µL of 2YT culture medium. 50 µL of the secondarily diluted product was evenly spread on a LB plate containing ampicillin and cultured at 37 °C overnight.
7) The next day, the plate was taken out to count the number of clones produced by each ligation and calculate the library capacity.
8) Simultaneously, 20 monoclonal colonies from the plate were picked and cultured in 2YT culture medium containing ampicillin at 37 °C for approximately 6-8 hours. The bacterial suspension was then subjected to sequencing by using universal primer M13R, in order to determine the diversity of the library.

### 7. Selection of the Phage Display Library

The steps for resuscitation and rescue of the phage display library and phage precipitation were as follows:
1) The electrotransformation product was diluted with 2YT to adjust the OD600 to approximately 0.2. Ampicillin with a final concentration of 100 ug/mL was added, and the mixture was incubated in a constant temperature shaker at 37 °C and 225 rpm until the OD600 reached 0.5;
2) M13KO7 was added. After thorough mixing, the mixture was allowed to stand at 37 °C for 30 minutes and then cultured at 37 °C and 225 rpm for 1 hour.
3) Volume of M13KO7 = 10 × Volume × OD600 × 5 × 108 / Titer of M13KO7
4) The bacterial suspension was centrifuged at 6000 rpm for 10 minutes and resuspended in 2YT-AK medium. It was then cultured overnight at 25 °C and 200 rpm;
5) The bacterial suspension was centrifuged at 10000 rpm for 15 minutes;
6) The precipitate was discarded, and the supernatant was transferred to a new centrifuge tube. PEG/NaCl with a volume of 1/5 bacterial suspension was added to the tube, mixed thoroughly, and the mixture was placed at 4 °C for 2 hours.
7) The precipitated phage supernatant was centrifuged at 10000 rpm, 4 °C, for 30 minutes. The supernatant was discarded, and the precipitate (phage) in each 50 ml centrifuge tube was resuspended in 1ml of sterile PBS.
8) The resuspended phage was transferred to a 1.5 mL EP tube and centrifuged at 12000 g, 4 °C, for 5 minutes.
9) The supernatant was transferred to a new 1.5 ml EP tube, and 250 µl of PEG/NaCl was added to each tube. After thorough mixing, the tubes were placed at 4 °C for 10 minutes.
10) Centrifugation was performed at 12000g for 10 minutes. The supernatant was discarded, and 1ml of PBS was added for resuspension.
11) Centrifugation was performed at 12000g for 5 minutes. The precipitate was discarded, and the supernatant was transferred to a new 1.5 ml EP tube.
12) Centrifugation was repeated at 12000g for 5 minutes, and the supernatant was transferred to a new 1.5ml EP tube, thereby obtaining the original phage library.
13) 10µl of the precipitate was added to 90µl of 2YT medium, labeled as 10⁻¹, and serially 10-fold diluted to 10⁻⁹. 20µl of the gradient diluted samples from the 10⁻⁷, 10⁻⁸, and 10⁻⁹ were added to 200 µl of pre-prepared ER2738 with an OD600 of 0.5. The mixture was thoroughly mixed and placed in 37 °C water bath for 10 minutes. Each 108 µl of the mixture was spread onto an LB-AMP solid plate and incubated overnight at 37 °C. The number of plaques was counted the next day to determine the titer.
14) Calculation of titer: Plates with a plaque number between 30-300 were selected. Titer was obtained by taking the average of two plates, multiplying the plaque number by the dilution fold and then multiplying by 100.

### 8. Panning of the Phage Display Library

The solid-phase panning process for the phage display library was as follows:
ELISA plates were coated with the target protein, after several wash steps, the recombinant phages bound to the immobilized antigen were eluted using TEA and amplified. After 3-4 rounds of panning, monoclonal colonies were selected for sequencing.
1) The antigen was diluted with PBS to 50 µg/ml, and 150 µl/well of antigen was added to coat a total of 3 wells. The plates were incubated overnight at 4 °C;
2) The target protein was removed, and the wells were blocked with 3% MPBS for 1 hour at room temperature;
3) The lib phage or the precipitate from the previous round of amplification was diluted with 450µl of 3% MPBS. 6×10¹¹ Pfu of phage was added to the wells coated with control protein (2×10¹¹ Pfu per well). 150 µl of the diluted phage was added to each well and incubated for 1 hour at room temperature;
4) The MPBS in target protein wells was discarded, and the phage from control wells was transferred to the wells coated with target protein for incubation for 1-1.5 hours at room temperature;
5) The phage was discarded, and the wells were washed 8-10 times with 0.05% PBST for 2-3 minutes each time, followed by 4-5 washes with PBS for 2-3 minutes each time. Simultaneously, the 1.5ml EP tubes that had been blocked previously were washed with PBS;
6) The phages were eluted using 1×TEA at 200 µl per well for 6-8 minutes. The eluted products were collected into the pre-blocked EP tubes, and 100 µl of Tris-HCl was added to each well for neutralization.
7) 10 µl of the output product was added to 90 µl of 2YT medium, labeled as 100, and subsequently diluted 10-fold until 10⁻². 20µl of the gradient diluted samples from 10¹, 10⁰, 10⁻¹, and 10⁻² were added to 200µl of pre-prepared ER2738 with an OD600 of 0.5 (10¹ referred to directly adding 20µl of the undiluted product to ER2738). The mixture was thoroughly mixed and placed in a 37 °C water bath for 10 minutes. Each 108 µl of the mixture was spread onto an LB-AMP solid plate and incubated overnight at 37 °C. The number of plaques was counted the next day to determine the titer.
8) Titer calculation: Plates with a plaque number between 30-300 were selected. The titer was calculated by taking the average of two plates, multiplying the number of plaques by the dilution fold and the elution volume.

The cell-based panning process for the phage display library was conducted as follows:
The recombinant cell lines overexpressing the target protein were used. The phage library was sequentially incubated with empty cells and cells overexpressing the target protein. After several washes to remove non-specifically binding phages, the recombinant phages bound to the cell surface were eluted by glycine or TEA and then amplified. After 3-4 rounds of panning, monoclonal colonies were selected for ELISA detection.
1) 1.5ml EP tubes were blocked with 1% PBSA one day in advance, and left at 4°C overnight;
2) 1×10⁷ of target cells and control cells were taken, washed three times with PBS, resuspended in 10mL of 1% PBSA, and placed on a decolorization shaker for low-speed blocking at room temperature for 1 hour, respectively;
3) 1×10¹¹ of phages were added to the control cells and incubated for 1 hour, while the target cells were continued to be blocked;
4) The incubated cells were centrifuged at 1000g for 5 minutes. The supernatant of the target cells (1% PBSA) was discarded, and the supernatant of the control cells was carefully aspirated and added to the target cell tube. After resuspension, the cells were placed on a shaker for low-speed incubation for 1 hour;
5) The target cell was centrifuged at 1000g for 5 minutes (while 1.5 ml EP tubes blocked one day in advance were washed three times with PBS). The supernatant of the target cells was discarded, 4ml of PBS was added for resuspension, and the suspension was distributed into closed 1.5ml EP tubes. The cells were centrifuged and washed five times with PBS, then transferred to four additional EP tubes for further 5 washes. Finally, the cells were transferred to the same EP tube;
6) The cells were resuspended in 200µl of PBS, and then 200µl of 2xTEA was added and immediately blown until the solution was not viscous. 200µl of Tris-HCl was then added for neutralization, thereby obtaining the final product.
7) The titer of the Output library was determined by the same protocol as for solid-phase panning.

### 9. Phage ELISA

1) 2YT-Amp medium was dispensed into a 96-well deep-well plate with 500 µl per well. Mono clones from the output plate were picked into the wells and cultured at 37°C, 225 rpm until the OD600 reached 0.5. The last two wells, H11 and H12, were filled with only medium, serving as blank controls without clones;
2) Concurrently, the ELISA plate was coated with antigen using CBS at a concentration of 1 µg/ml, with 100 µl per well, and incubated at 37°C for 2 hours.
3) 2YT-Amp medium was dispensed into an additional 96-well deep-well plate with 500 µl per well. 10 µl of bacterial suspension with OD600 of 0.5 was transferred into the newly dispensed 96-well plate by using a multi-channel pipette, and cultured overnight at 37°C, 225 rpm. This bacterial suspension was for sample sequencing;
4) M13KO7 was added to the bacterial suspension with OD600 of 0.5, mixed thoroughly, and allowed to stand at 37°C for 15 minutes;
5) The volume of M13KO7 = 10 ×volume × OD600 × 5 × 108 / M13KO7 titer.
6) The bacterial suspension after infection was placed on a shaker and cultured at 37°C, 225 rpm for 45 minutes.
7) The bacterial suspension was centrifuged at 4000 rpm for 10 minutes in a centrifuge. The supernatant was discarded, and the bacteria were resuspended in 800 µl per well of 2YT-AK medium. The plate was then placed back on the shaker and cultured overnight at 30°C, 210 rpm.
8) Concurrently, the antigen in the ELISA plate was removed, and the plate was washed three times with PBST wash buffer and blocked with 250 µl per well of 3% MPBS at 4°C overnight. An additional blank plate was also blocked as a blank control.
9) On the second day, the 96-well deep-well plate was centrifuged at 4000 rpm for 10 minutes in a centrifuge. The milk in the ELISA plate was discarded, and the plate was washed four times with 200 µL PBST. 50 µl of PBST was added to each well, followed by the addition of 50 µL of centrifuged phage supernatant corresponding to each well. The plate was incubated at 4°C for 1 hour. The supernatant was discarded, and the plate was washed five times with PBST. HRP-Anti M13 secondary antibody was diluted with PBST and added to each well at 100 µl. After incubation at 4°C for 45 minutes, the secondary antibody was washed away. The plate was washed five times with PBST. TMB was added for color development at room temperature for 10 minutes, followed by termination with hydrochloric acid. The plate was read, and clones with high S/N ratios were selected for sequencing using the preserved bacterial suspension.

### 10. Amplification, Transient Transfection, and Detection of Overlap PCR Products

### 10.1 First Round of PCR: Amplification of CMV, VHH, and FC

1) The PCR reaction system was configured [50 µL system/reaction] (additional CMV and FC fragments could be prepared). (Primers for CMV fragment amplification: CMV-F and PCom-R1; primers for VHH fragment amplification: PCom-F1 and PCom-R2; primers for FC fragment amplification: PCom-F2 and PGK-R)
   Components and quantities: forward primer (5 µM) 2 µL, reverse primer (5 µM) 2 µL, NuHi Power mix (2×) 25 µL, template (plasmid or bacterial suspension) 1 µL, sterile water added to 50 µL.
   The PCR reaction program was as follows: 95°C for 10 minutes; (95 °C for 15 seconds; 56 °C for 30 seconds; 68 °C for 60 seconds) × 25 cycles; 68°C for 10 minutes.
2) 50 µL of PCR product was taken, and 10× loading buffer with a volume of 1/10 was added. Electrophoresis analysis was performed using 1% agarose. The sizes for CMV and FC bands were approximately 750 bp, and the size for VHH band was approximately 560 bp.
3) The target bands were excised from the gel. The PCR products were purified and subjected to the determination of concentration by using NanoDrop (if the concentration was too high, it could be diluted before subsequent reactions).

### 10.2 Second Round of PCR: Overlap Extension PCR to Connect CMV, VHH, and FC

1) The PCR reaction system was configured:
   Components and quantities: CMV 1st product 50 ng, VHH 1st product 50 ng, FC 1st product 50 ng, NuHi Power mix (2×) 25 µL, sterile water added to 46 µL.
   The PCR reaction program was as follows:
      95 °C for 10 minutes; (95 °C for 15 seconds; 60 °C for 30 seconds; 68 °C for 120 seconds) × 15 cycles.
      2 µL of each primer CMV-F and PGK-R were added.
      The PCR reaction program was as follows:
         95 °C for 10 minutes; (95 °C for 15 seconds; 60 °C for 30 seconds; 68 °C for 120 seconds) × 20 cycles; 68°C for 10 minutes.
4) The overlap PCR product was purified using TakaRa DNA fragment recovery kit and subjected to the determination of concentration by using NanoDrop. At least 10 µg of PCR product was required for subsequent cell transfection and verification.
5) The transfection steps were the same as those for the transfection of eukaryotic expression vectors.

### 11. Transient Transfection and Expression of Single Domain Antibodies

1. LVTransm transfection reagent and antibody expression vector pcDNA3.4-hIgG1-Fc2 or overlap PCR product were taken from the refrigerator, thawed at room temperature, and then blown up and down with a pipette until well mixed. PBS buffer was taken out and warmed to room temperature. 500 µL of PBS was taken into one well of a 24-well plate and added with 4 µg pcDNA3.4-hIgG1-Fc2, blown up and down with a pipette until well mixed, then added with 12 µL of LVTransm and immediately blown up and down with a pipette until well mixed. Then the plate was placed at room temperature for 10 minutes. The mixture here was called DNA/LVTransm complex.
2. The above 532µL of DNA/LVTransm complex was added into 1.5 mL of 293F cells, gently shaken until well mixed. The cells were placed in a 37°C, 5% CO₂ incubator and cultured at 130 RPM for 6-8 hours. Then 1.5 mL of fresh 293 culture medium was added, and the cells were put back to the incubator for further cultivation.
3. After 3 days of continuous culture, the culture supernatant was collected and filtered with a 0.45 µm filter membrane, and the filtrate was transferred to a sterile centrifuge tube for following flow cytometry and ELISA detection.

### 12. Construction of Eukaryotic Expression Vectors

Based on the Elisa detection results of phage mono clones, positive clones were selected for sequencing to obtain VHH antibody sequences. The obtained VHH antibody sequences were analyzed and synthesized, and subcloned in tandem with human IgG1Fc into the expression vector pcDNA3.4-hIgG1-Fc2. After the vectors were sequenced and confirmed to be correct, endotoxin-free plasmids were prepared by using Qiagen plasmid extraction kit for later use.

### 13. Flow cytometry detection of recombinant antibodies binding to target proteins

1. CHO-S cells and CHO-S/UPAR cell strains were thawed from liquid nitrogen and the cell state was adjusted to logarithmic growth phase.
2. Each of the two kinds of cells was divided into several sections with 5*10^5 cells in each section.
3. The expressed antibodies were incubated with target cells respectively by mixing well, and incubating at room temperature for 1 hour.
4. The cells were centrifuged at 800xg at room temperature for 5 minutes, the supernatant containing antibodies was discarded, and the cells were washed with PBS for 3 times.
5. 1 µl of PE-labeled Anti-Human IgG was added, mixed well, and incubated at room temperature in dark for 30 minutes.
6. The cells were centrifuged at 800xg at room temperature for 5 minutes, the supernatant containing the secondary antibodies was discarded, and the cells were washed with PBS for 3 times.
7. The cells were resuspended with 500 µL of PBS, and then subjected to flow cytometry.

### 14 Expression and purification of single domain antibodies

1. LVTransm transfection reagent and single chain antibody expression vectors were taken from the refrigerator, thawed at room temperature, and then blown up and down with a pipette until well mixed. PBS or HBSS buffer was taken out and warmed to room temperature. 2mL of PBS was taken into one well of a 6-well plate and added with 130 µg pcDNA3.4-hIgG1-Fc2, blown up and down with a pipette until well mixed, then added with 400 µL of LVTransm and immediately blown up and down with a pipette until well mixed. Then the plate was placed at room temperature for 10 minutes.
2. The above DNA/LVTransm complex was added into 50 mL of 293F cells, gently shaken until well mixed. The cells were placed in a 37°C, 5% CO₂ incubator and cultured at 130 RPM for 6-8 hours. Then 50 mL of fresh 293 culture medium was added, and the cells were put back to the incubator for further cultivation.
3. After 7 days of continuous culture, the culture supernatant was collected and filtered with a 0.45 µm filter membrane, and the filtrate was transferred to a sterile centrifuge tube. The antibody was purified using a Protein A column.

### 15. Affinity Detection of Recombinant Antibody

UPAR recombinant protein was immobilized on a CM5 chip using 10 mM Acetate buffer, and the prepared single domain antibodies were used as the mobile phase to detect the binding ability of the candidate single domain antibodies to the target protein UPAR.

### Example 1: Preparation and Purification of PD1 Single Domain Antibodies

### 1.1 Detection of PD1-Fc Recombinant Protein Activity

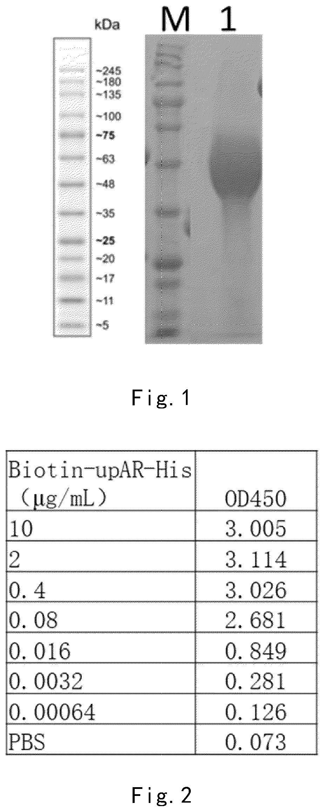

UPAR-His antigen was prepared through Experimental Method 1 described above, and the expression of UPAR-His protein was detected by SDS-PAGE. The results are shown in Fig. 1. Lane 1 shows the UPAR-His protein band with a theoretical molecular weight of 32.3 kDa and but detected as approximately 45 kDa by SDS-PAGE.

The ELISA plate was coated with 2µg/ml of uPA (Novoprotein, C393). Five-fold gradient diluted biotinUPAR-His was used as the primary antibody, and streptavidin-HRP was used as the secondary antibody.

**Results:** According to the ELISA detection results, UPAR-His can bind to its natural ligand uPA (Fig. 2), indicating its activity and suitability for llama immunization and antibody screening.

### 1.2 Detection of Immunological Potency by ELISA

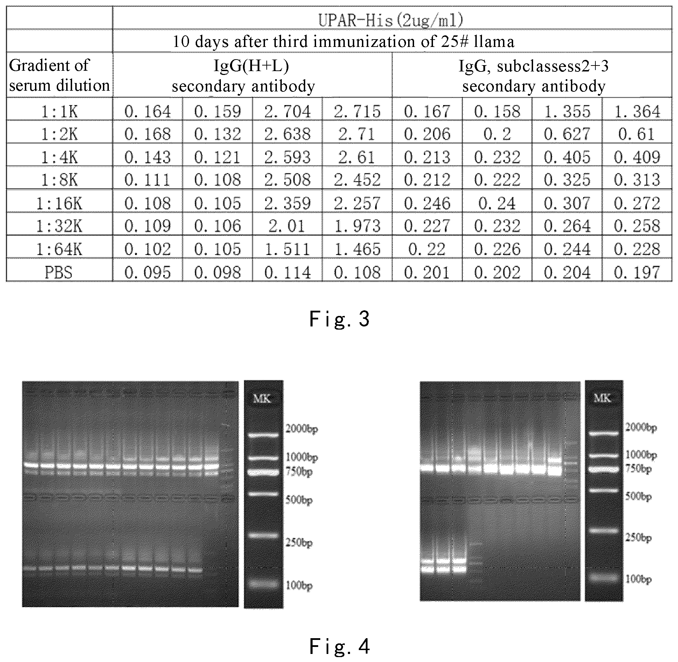

Llamas were immunized through Experimental Method 2 described above. The immunological potency was detected through Experimental Method 3. Specifically, serum was isolated from the llamas after the third immunization, diluted according to the dilution gradient shown in Fig. 3, and subjected to ELISA detection using 96-well plates pre-coated with UPAR-His antigen.

**Results:** According to the ELISA detection results (Fig. 3), the immune serum bound to UPAR-His with an OD450 value exhibiting gradient change along with the gradient dilution of serum, indicating successful immunization. Detection using IgG, subclasses 2+3 as the secondary antibody also showed high immune potency. 100 mL of peripheral blood was collected for library construction.

### 1.3 Amplification of VHH Fragments

PBMC isolation and VHH fragment amplification were performed through Experimental Methods 4 and 5 described above. Specifically, total RNA was extracted from peripheral blood of the llamas immunized in Example 1.2, reverse transcribed into cDNA, and subjected to two rounds of nest PCR by using primers for single domain antibody amplification.

Results: Fig. 4 shows the result of agarose gel electrophoresis of PCR products. The first round of PCR yielded two PCR bands of approximately 1000bp and 750bp, and the 750bp fragment was recovered from the gel and used as the template for the second round of PCR. The second round of PCR yielded a band of approximately 450bp, which is the VHH fragment.

### 1.4 Diversity Analysis of the Phage Display Library

The construction and diversity analysis of the phage display library were carried out through Experimental Method 6 described above. Specifically, the VHH fragments obtained by the amplification in Example 1.3 were digested with SfiI enzyme and subcloned into the phage display vector pComF. The connected products were then electrotransformed into SS320 *E. coli* competent cells to construct the single domain antibody phage display library. The capacity of the phage display library was calculated as 2.53E8. From the phage display library, 20 mono clones were randomly selected for sequencing to analyze the diversity of the phage display library.

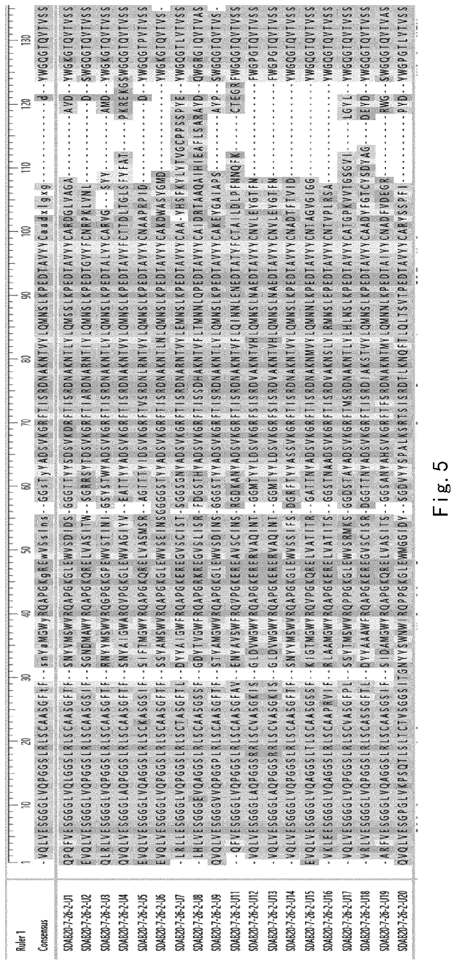

**Results:** As shown in Fig. 5, sequence alignment revealed that the empty vector rate and antibody repetition rate of the phage display library were no higher than 10%.

### 1.5 Solid-Phase Panning of the Phage Display Library

Solid-phase panning of the phage display library was performed through Experimental Method 7 described above. Specifically, coating with UPAR-His and Fc recombinant proteins respectively, and the constructed phage display library underwent four rounds of screening and enrichment to enrich positive clones.

### 1.6 Phage ELISA of the Products from Solid-Phase Panning of the Phage Display Library

From the enriched phage positive clones in Example 1.5, phage mono clones were selected and identified by Phage ELISA through Experimental Method 8 described above.

Specifically, the first, second and third round outputs from solid-phase panning were chosen for Phage ELISA experiments. Wells of the plate were coated with UPAR-His antigen protein respectively for phage Elisa detection. Control groups were directly blocked wells and Fc-coated wells.

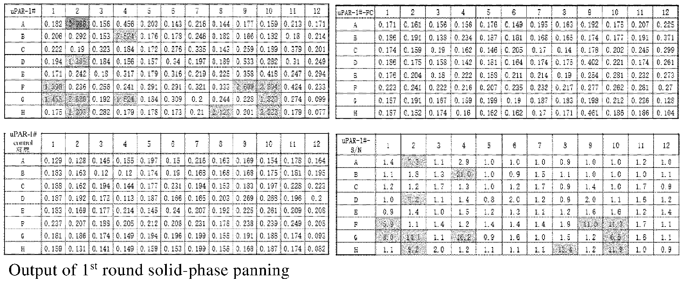

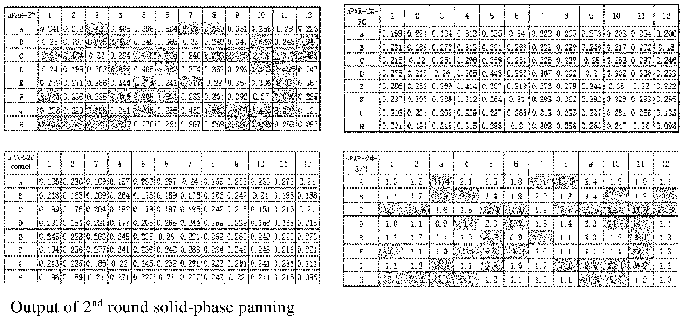

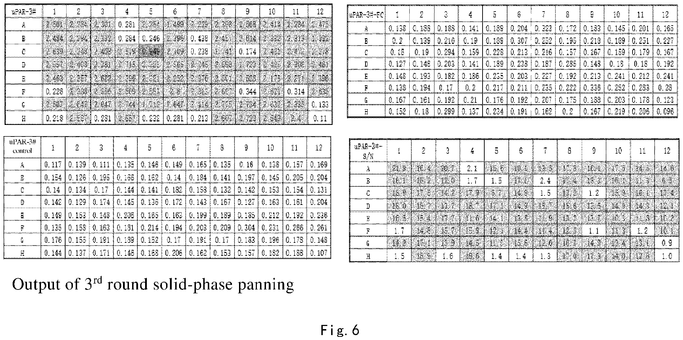

**Results:** The result of phage ELISA detection is shown in Fig. 6. Clones with an S/N ratio above 5 and not binding to Fc were selected for sequencing and analysis of sequence antibodies. 18 different antibodies were obtained by solid-phase panning of antibodies.

### 1.7 Flow Cytometry Detection of Transient Transfection Product of Overlap product

In this example, vector construction and expression detection were performed on the 18 VHH antibody sequences screened and sequenced in Example 1.6. CMV promoter, signal peptide, and human IgG1 Fc tag were added to the N- and C-terminus of the candidate antibody sequences through Overlap PCR. The purified PCR products were transiently transfected into 293 cells to express the antibodies for flow cytometry detection.

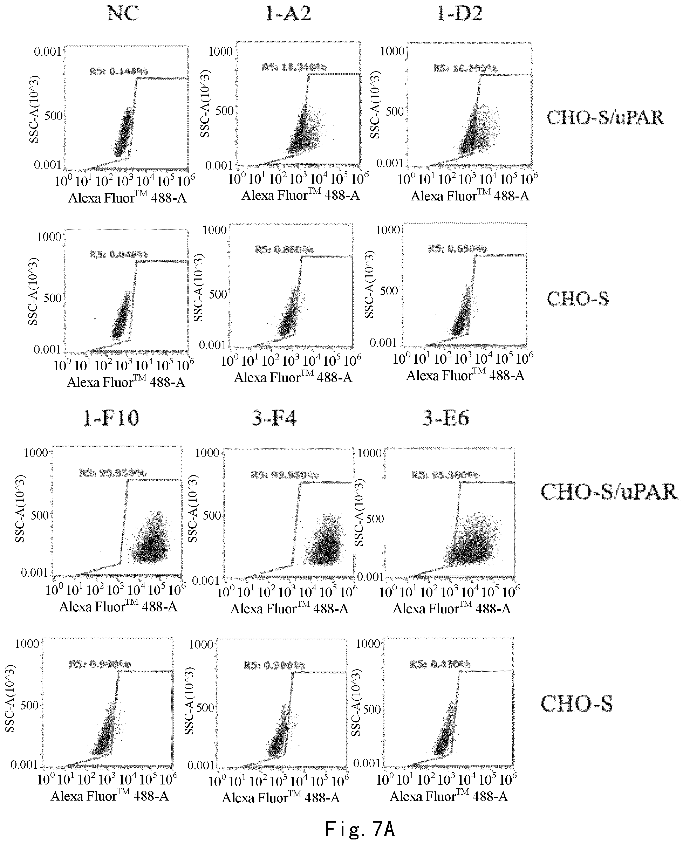

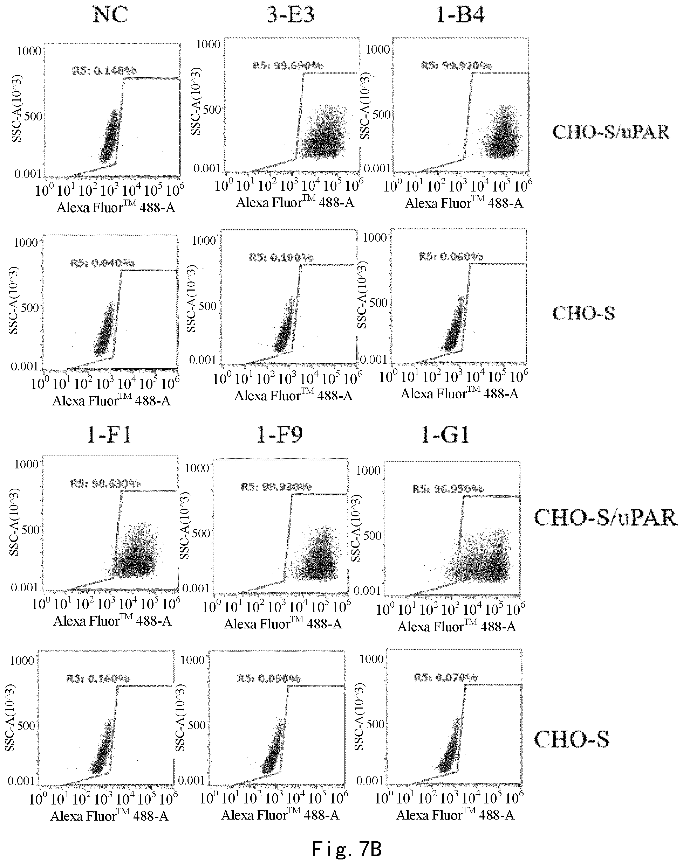

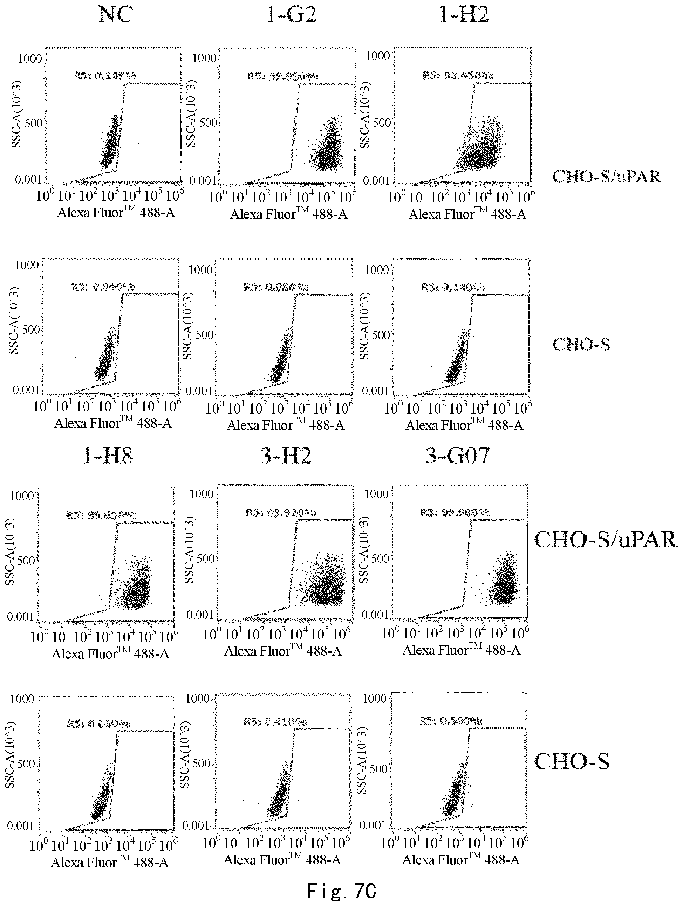

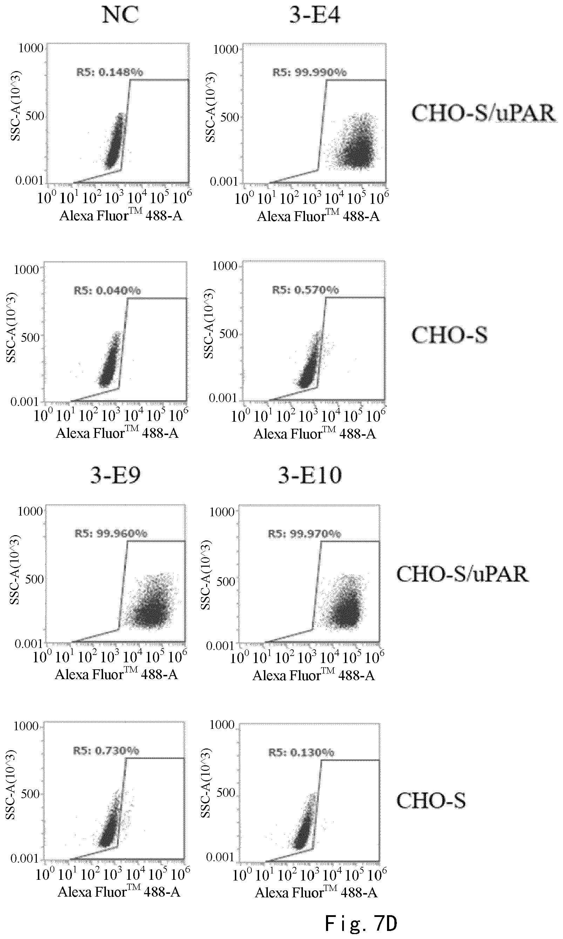

**Results:** The FACS detection results are shown in Fig. 7A-7D. Among the 18 clones obtained from phage display library panning, 15 suitable clones were selected for synthesis of expression vectors. Two clones (1-A2 and 1-D2) that showed very weak binding to the UPAR-overexpressing cell line, and a clone (3-F4) with a high similarity to known sequences, were not used for synthesis of expression vectors.

### 1.8 Expression, Purification, and Flow Cytometry Analysis of Candidate Antibodies

The candidate antibody expression vectors were transiently transfected into HEK293 cells. The expressed antibodies in the supernatant were purified using protein A, and subjected to protein quantification and SDS-PAGE analysis. The 15 candidate single domain antibodies were subjected to flow cytometry analysis with the overexpressing cell line CHO-S/UPAR and its parental cell line.

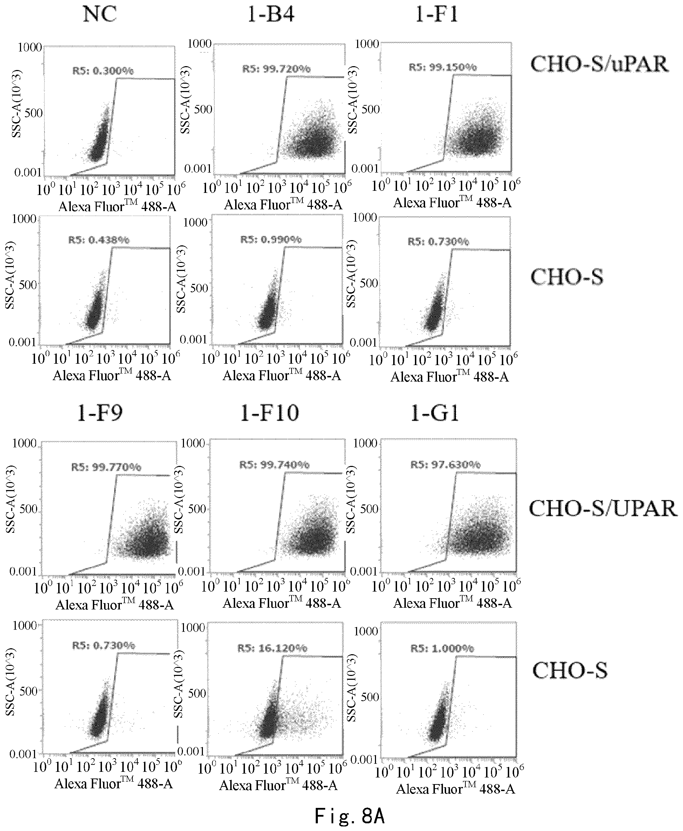

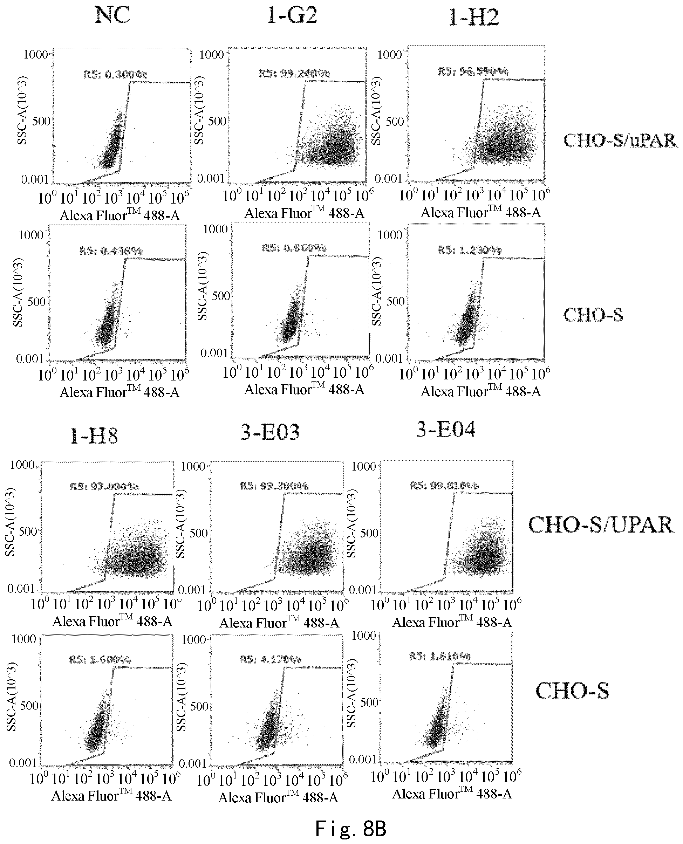

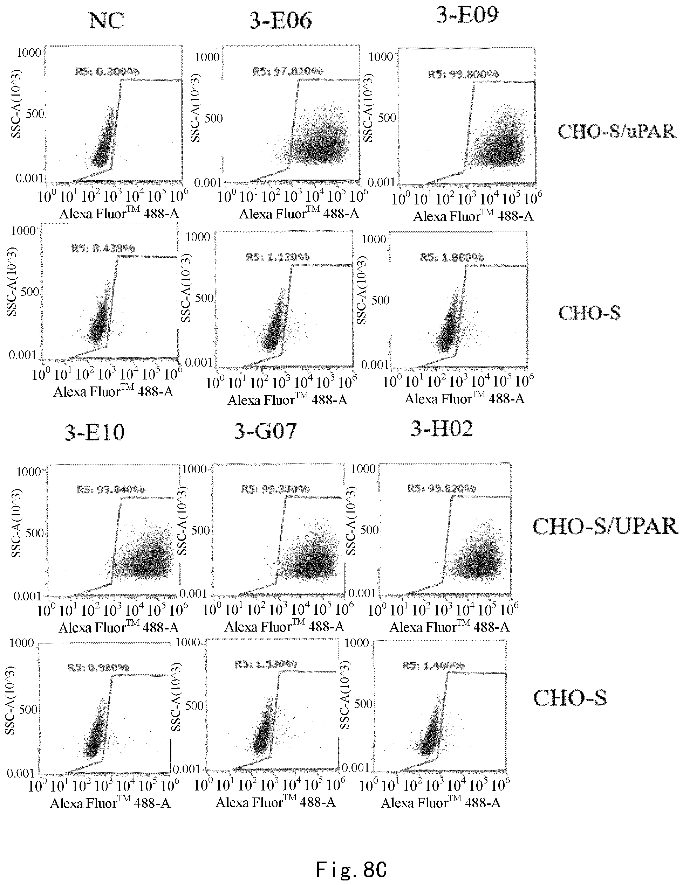

**Results:** The flow cytometry analysis indicated that all 15 candidate single-domain antibodies are able to specifically bind to CHO-S/UPAR (Figs. 8A-8C).

The sequences obtained from sequencing the 15 antibodies are as follows:
1) pcDNA3.4-SDAB20-7-26-2-1-B4-hFC 
QVQLVESGGGLVQPGGSLRLSCAASGFTLDYYAIGWFRQAPGKEREGVSC
ISSTGGSTNYADSVKGRFTISRDNAKNTVYLQMNSLKPEDTAVYYCAADTHYL
 TVCYDRLGFDYWGQGTQVTVSS (SEQ ID NO 1);
2) pcDNA3.4-SDAB20-7-26-2-1-F1-hFC 
EVQLAESGGGLVQPGGSLRLSCAASGFTFSSYTMSWVRQAPGKGLEWVS
AIDSDGGVTSYADSVKGRFTISRDNAKNTLYLQMNSLKPEDTAVYYCANGGYC
SGYGCYPRLLSQGTLVTVSS (SEQ ID NO 2);
3) pcDNA3.4-SDAB20-7-26-2-1 -F9-hFC 
QSLLVESGGGLVQPGGSLRLSCAASGFSLDYYAIGWFRQAPGKEREEVSCI
SSGGGSTNYADSVKGRFTISRDNAKNTVYLQMNSLKPEDTAVYYCAAKRNFM
LYLVQCPYEYDYWGQGTQVTVSS (SEQ ID NO 3);
4) pcDNA3.4-SDAB20-7-26-2-1 -F 10-hFC 
QVQLVESGGGLVQPGGSLRLSCAASGSILSIRAVGWYRQAPGKQRELVALI
TSDGTTHYALSVKGRFTISRDNAKTTVYLQMNSLKPEDTAVYYCNREDYARRY
WGQGTQVTVSS (SEQ ID NO 4);
5) pcDNA3.4-SDAB20-7-26-2-1-G1-hFC 
QVQLVESGGGLVQPGGSLRLSCAASGNSLRRYAIGWFRQAPGKEREGVVC
ISTVGGSSYYVDSVKGRFTISEDNVKNTAYLQMNSLEPEDTAVYYCAAANRLY
CPAYGSAGYDYWGQGTQVTVSS (SEQ ID NO 5);
6) pcDNA3.4-SDAB20-7-26-2-1 -G2-hFC 
QSQLAESGGGLVQPGGSLRLSCVASRFSLDAYAIAWFRQAPGKEREGVSCI
SSIGGRTNYADPVKGRFTISRDNAKNTAYLEMNSLQPEDTAVYYCAAVQRLFG
PCLLSGGMDYWGKGTPVTVSS (SEQ ID NO 6);
7) pcDNA3.4-SDAB20-7-26-2-1-H2-hFC 
QVQLVESGGGLVQPGGSLRLSCAASGSTLDYYAIAWFRQAPGKGREGVSC
ISSSGGRTNYADSVKGRFTVSRDNVKNTVYLQMNSLKPEDTAVYNCAAGGDLS
CYGTPTSIWQYDLWGQGTQVTVAS (SEQ ID NO 7);
8) pcDNA3.4-SDAB20-7-26-2-1-H8-hFC 
QRQLVESGGGLVQPGGSLRLSCAVSEFTLDYYAIGWFRQAPGKEREGVSCI
SSSGGSTVYADSVKGRFTISADNAKNTVYLQMNSLKPEDTAVYYCAAGSLLLF
RLCVSRLYEYDYWGQGTQVTVSS (SEQ ID NO 8);
9) pcDNA3.4-SDAB20-7-26-2-3-E03-hFC 
AVQLVDSGGGLVQAGGSLRLSCAASGIRLSSNAMAWYRQAPGKQRGLVA
LITSDGTTNYVDSVKGRFTISRDNAKNTVYLQMNSLKPEDTAVYYCKREPYLST
RGYWGQGTQVTVSS (SEQ ID NO 9);
10) pcDNA3.4-SDAB20-7-26-2-3-E04-hFC QLRLVESGGGLVQPGGSLRLSCAASGFTLDYYDIGWFRQAPGKEREGVSCI 
GRISGDTDYAESVKGRFTASRDNAKNTVYLQMNSLKPEDTAVYYCAAERRFFG
GGCRRSVDNMDSWGKGTLVTVSS (SEQ ID NO 10);
11) pcDNA3.4-SDAB20-7-26-2-3-E06-hFC 
EVQLVESGGGLVQAGGSLTLSCVASASGSILRLNAMGWYRQAPAKQREL
VALITRDGSTNYPDSVKGRFTISRDNAKNTVYLQMSSLSPEDTAVYYCYVQNH
YSNYWGQGTQVTVSS (SEQ ID NO 11);
12) pcDNA3.4-SDAB20-7-26-2-3-E09-hFC 
AVQLVDSGGGLVQPGGSLRLSCAASGFTFSSYAMGWARQVPGKGLEWVS
IIYRDGNTYYTDSVKGRFTISRDNAKNTVYLQMNSLKPEDTAVYYCAPVETVA
ARRRDYWGQGTQVNVSS (SEQ ID NO 12);
13) pcDNA3.4-SDAB20-7-26-2-3-E10-hFC 
AVQLVDSGGGLVQPGGSLRLSCAASGFTFSSYAMGWARQVPGKGLEWVS
IIYRDGNTYYTDSVKGRFTISRDNAKNTVYLQMNSLKPEDTAVYYCAPVETVA
ARRRDYWGQGTQVNVSS (SEQ ID NO 13);
14) pcDNA3.4-SDAB20-7-26-2-3-G07-hFC 
RLQLVESGGGLVQPGGSLRLSCAVSGKALDYYSIGWFRQAPRKQREGVSC
ISSSGGSTNYADSVKGRFTISRDNAKDTVYLQMNSLKPEDTADYYCAVVSTVL
CGLGIYEYDYWGQGTLVTVSS (SEQ ID NO 14);
15) pcDNA3.4-SDAB20-7-26-2-3-H02-hFC

QLTLVESGGGLVQPGGSLRLSCAASEFTLDYYAIGWFRQAPGKEREGVSCI
SSSDGSTYYADSVKGRFTISRDNAKNTVYLQMNSLKPEDTAVYYCAIGGLLLLR
LWERGNYEFDYWGQGTQVTVSS (SEQ ID NO 15).

The CDR regions of each VHH chain are underlined.

The amino acid sequences of CDR regions of the antibodies are shown in Table 9.

**Table 9: amino acid sequences of the CDR regions of the antibodies of the present invention**

| Name of antibody | CDR | Sequence | SEQ ID NO |
|---|---|---|---|
| | CDR1 | GFTLDYYA | 16 |
| 1-B4 | CDR2 | ISSTGGST | 17 |
| | CDR3 | YYCAADTHYLTVCYDRLGFDY | 18 |
| | CDR1 | GFTFSSYT | 19 |
| 1-F1 | CDR2 | IDSDGGVT | 20 |
| | CDR3 | YYCANGGYCSGYGCYPRL | 21 |
| 1-F9 | CDR1 | GFSLDYYA | 22 |
| | CDR2 | ISSGGGST | 23 |
| | CDR3 | YYCAAKRNFMLYLVQCPYEYDY | 24 |
| | CDR1 | GSILSIRA | 25 |
| 1-F10 | CDR2 | LITSDGTT | 26 |
| | CDR3 | YYCNREDYARRY | 27 |
| | CDR1 | GNSLRRYA | 28 |
| 1-G1 | CDR2 | ISTVGGSS | 29 |
| | CDR3 | YYCAAANRLYCPAYGSAGYD | 30 |
| | CDR1 | RFSLDAYA | 31 |
| 1-G2 | CDR2 | ISSIGGRT | 32 |
| | CDR3 | YYCAAVQRLFGPCLLSGGMDY | 33 |
| | CDR1 | GSTLDYYA | 34 |
| 1-H2 | CDR2 | ISSSGGRT | 35 |
| | CDR3 | YNCAAGGDLSCYGTPTSIWQYDL | 36 |
| | CDR1 | EFTLDYYA | 37 |
| 1-H8 | CDR2 | ISSSGGST | 38 |
| | CDR3 | YYCAAGSLLLFRLCVSRLYEYDY | 39 |
| | CDR1 | GIRLSSNA | 40 |
| 3-E03 | CDR2 | LITSDGTT | 26 |
| | CDR3 | YYCKREPYLSTRGY | 41 |
| | CDR1 | GFTLDYYD | 42 |
| 3-E04 | CDR2 | IGRISGDT | 43 |
| | CDR3 | YYCAAERRFFGGGCRRSVDNMDS | 44 |
| | CDR1 | ASASGSILRL | 45 |
| 3-E06 | CDR2 | LITRDGST | 46 |
| | CDR3 | YYCYVQNHYSNY | 47 |
| | CDR1 | GFTFSSYA | 48 |
| 3-E09 | CDR2 | IIYRDGNT | 49 |
| | CDR3 | YYCAPVETVAARRRDY | 50 |
| | CDR1 | GFTFSSYA | 48 |
| 3-E10 | CDR2 | IIYRDGNT | 49 |
| | CDR3 | YYCAPVETVAARRRDY | 50 |
| | CDR1 | GKALDYYS | 51 |
| 3-G07 | CDR2 | ISSSGGST | 38 |
| | CDR3 | YYCAVVSTVLCGLGIYEYDY | 52 |
| | CDR1 | EFTLDYYA | 37 |
| 3-H02 | CDR2 | ISSSDGST | 53 |
| | CDR3 | YYCAIGGLLLLRLWERGNYEFDY | 54 |

### Example 2: Detection of Antibody Affinity

Three representative antibodies (1-F09, 3-E04, 3-H02) with the highest binding rates were selected and subjected to antibody affinity detection using the aforementioned Experimental Method 15.

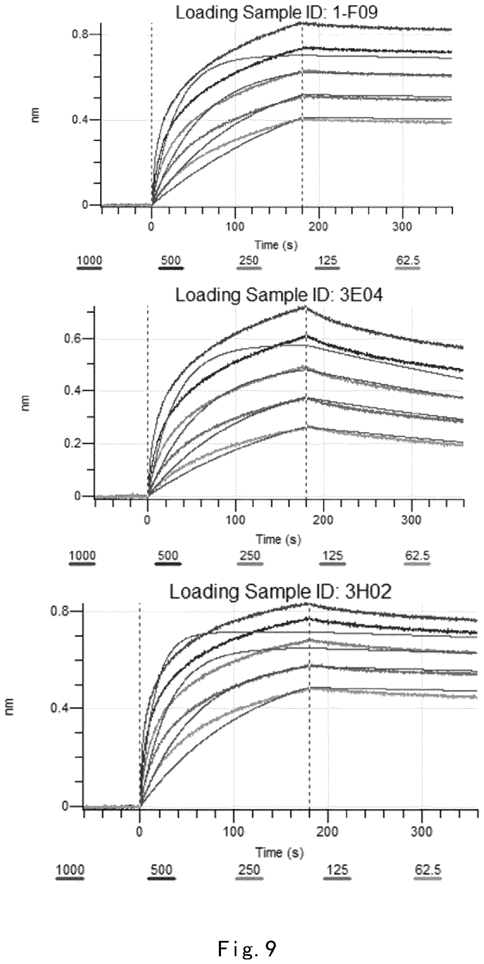

**Results:** As shown in Fig. 9 and Table 10.

**Table 10: Detection Results of Recombinant Antibody Affinity**

| | Kon | Koff | KD |
|---|---|---|---|
| 1-F09 | 6.764×10⁴ M⁻¹S⁻¹ | 1.008×10⁻⁴ S⁻¹ | 1.49×10⁻⁹ M |
| 3-E04 | 6.268×10⁴ M⁻¹S⁻¹ | 1.395×10⁻³ S⁻¹ | 2.226×10⁻⁸ M |
| 3-H02 | 1.322×10⁵ M⁻¹S⁻¹ | 1.731×10⁻⁴ S⁻¹ | 1.310×10⁻⁹ M |

This example demonstrates that the three representative antibodies of the present invention, 1-F09, 3-E04 and 3-H02, possess high affinity to UPAR.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. An anti-UPAR single domain antibody, wherein complementarity determining regions (CDRs) of a VHH chain of the anti-UPAR single domain antibody is one or more selected from the group consisting of:
(1) CDR1 as shown in SEQ ID NO: 37, CDR2 as shown in SEQ ID NO: 53, and CDR3 as shown in SEQ ID NO: 54;
(2) CDR1 as shown in SEQ ID NO: 22, CDR2 as shown in SEQ ID NO: 23, and CDR3 as shown in SEQ ID NO: 24;
(3) CDR1 as shown in SEQ ID NO: 42, CDR2 as shown in SEQ ID NO: 43, and CDR3 as shown in SEQ ID NO: 44;
(4) CDR1 as shown in SEQ ID NO: 25, CDR2 as shown in SEQ ID NO: 26, and CDR3 as shown in SEQ ID NO: 27;
(5) CDR1 as shown in SEQ ID NO: 28, CDR2 as shown in SEQ ID NO: 29, and CDR3 as shown in SEQ ID NO: 30;
(6) CDR1 as shown in SEQ ID NO: 31, CDR2 as shown in SEQ ID NO: 32, and CDR3 as shown in SEQ ID NO: 33;
(7) CDR1 as shown in SEQ ID NO: 34, CDR2 as shown in SEQ ID NO: 35, and CDR3 as shown in SEQ ID NO: 36;
(8) CDR1 as shown in SEQ ID NO: 37, CDR2 as shown in SEQ ID NO: 38, and CDR3 as shown in SEQ ID NO: 39;
(9) CDR1 as shown in SEQ ID NO: 40, CDR2 as shown in SEQ ID NO: 26, and CDR3 as shown in SEQ ID NO: 41;
(10) CDR1 as shown in SEQ ID NO: 19, CDR2 as shown in SEQ ID NO: 20, and CDR3 as shown in SEQ ID NO: 21;
(11) CDR1 as shown in SEQ ID NO: 45, CDR2 as shown in SEQ ID NO: 46, and CDR3 as shown in SEQ ID NO: 47;
(12) CDR1 as shown in SEQ ID NO: 48, CDR2 as shown in SEQ ID NO: 49, and CDR3 as shown in SEQ ID NO: 50;
(13) CDR1 as shown in SEQ ID NO: 51, CDR2 as shown in SEQ ID NO: 38, and CDR3 as shown in SEQ ID NO: 52; and
(14) CDR1 as shown in SEQ ID NO: 16, CDR2 as shown in SEQ ID NO: 17, and CDR3 as shown in SEQ ID NO: 18.

2. The anti-UPAR single domain antibody according to claim 1, wherein the amino acid sequence of the VHH chain of the anti-UPAR single domain antibody is selected from sequences shown in SEQ ID NOs: 1-15.

3. The anti-UPAR single domain antibody according to claim 1, wherein the amino acid sequence of the VHH chain of the anti-UPAR single domain antibody is selected from a sequence shown in SEQ ID NO: 3, 10 or 15.

4. The anti-UPAR single domain antibody according to claim 1, wherein the anti-UPAR single domain antibody is selected from the group consisting of: a humanized antibody, a camel-derived antibody, and a chimeric antibody.

5. An anti-UPAR antibody, which comprises one or more VHH chain(s) of the anti-UPAR single domain antibody according to claim 1.

6. The anti-UPAR antibody according to claim 5, wherein the amino acid sequence of the VHH chain of the anti-UPAR single domain antibody is selected from sequences shown in SEQ ID NOs: 1-15.

7. A polynucleotide encoding a protein selected from the group consisting of: the anti-UPAR single domain antibody according to claim 1, or the antibody according to claim 5.

8. An expression vector comprising the polynucleotide according to claim 7.

9. A host cell, wherein the host cell comprises the expression vector according to claim 8, or the genome thereof is integrated with the polynucleotide according to claim 7.

10. A method for producing an anti-UPAR single domain antibody, wherein the method comprises the steps of:
(a) culturing the host cell according to claim 9 under conditions suitable for producing a single domain antibody, thereby obtaining a culture containing the anti-UPAR single domain antibody;
(b) isolating and/or recovering the anti-UPAR single domain antibody from the culture; and
(c) optionally, purifying and/or modifying the anti-UPAR single domain antibody obtained in step (b).

11. An immunoconjugate which comprises:
(a) the anti-UPAR single domain antibody according to claim 1, or the anti-UPAR antibody according to claim 5; and
(b) a coupling moiety selected from the group consisting of: a detectable label, a drug, a toxin, a cytokine, a radionuclide, an enzyme, a gold nanoparticle/nanorod, a nanomagnetic particle, a viral coat protein or VLP, and a combination thereof.

12. A use of the anti-UPAR single domain antibody according to any one of claims 1-4, the anti-UPAR antibody according to claim 5, or the immunoconjugate according to claim 11 for preparing:
(1) a drug for preventing and/or treating a disease related to UPAR; and/or
(2) a reagent for detecting UPAR.

13. A pharmaceutical composition which comprises:
(a) the anti-UPAR single domain antibody according to claim 1, the anti-UPAR antibody according to claim 5, or the immunoconjugate according to claim 11; and
(ii) a pharmaceutically acceptable carrier.

14. A method of preventing and/or treating a disease related to UPAR, which comprises: administering the anti-UPAR single domain antibody according to claim 1, the anti-UPAR antibody according to claim 5, or the immunoconjugate according to claim 11, or the pharmaceutical composition according to claim 13, to a subject in need thereof.

15. The method according to claim 14, wherein the disease related to UPAR is selected from the group consisting of: an aging-related disease, a cancer or tumor.
